# EUROPEAN PATENT SPECIFICATION

(11) **EP 1 499 888 B1**
(45) Date of publication and mention of the grant of the patent: **07.07.2010**
(21) Application number: 03736501.2
(22) Date of filing: 29.04.2003
(51) Int. Cl.: G01N 33/53, G01N 33/567, A01N 37/18, A61K 38/00, C07K 1/00, C07K 14/00, C07K 17/00

(54) **CONFORMATIONALLY CONSTRAINED PEPTIDES THAT BIND THE ORL-1 RECEPTOR**
DEN ORL-1-REZEPTOR BINDENDE PEPTIDE MIT EINGESCHRÄNKTER KONFORMATION
PEPTIDES A CONFORMATION RESTREINTE SE LIANT AU RECEPTEUR ORL-1

(30) Priority: 29.04.2002 US 376745 P
(43) Date of publication of application: 26.01.2005
(60) Divisional application: 08152566.9
(73) Proprietor: EURO-CELTIQUE S.A., 1653 Luxembourg (LU)
(72) Inventor: KYLE, Donald, J., Newtown, PA 18940 (US)
(74) Representative: Neuefeind, Regina
(86) International application number: PCT/US2003/013097
(87) International publication number: WO 2003/093294

(56) References cited:
- BRAIN RESEARCH, vol. 905, 2001, pages 127-133, XP002341920 NL AMSTERDAM
- BRITISH JOURNAL OF PHARMACOLOGY., vol. 128, 1999, pages 1252-1258, XP002341921 GB BASINGSTOKE, HANTS.
- JOURNAL OF MEDICINAL CHEMISTRY., vol. 43, 2000, pages 2805-2813, XP002341922 US AMERICAN CHEMICAL SOCIETY. WASHINGTON.
- JOURNAL OF MEDICINAL CHEMISTRY., vol. 45, October 2002 (2002-10), pages 5280-5286, XP002341923 US AMERICAN CHEMICAL SOCIETY. WASHINGTON.
- JOURNAL OF MEDICINAL CHEMISTRY., vol. 44, 2001, pages 4015-4018, XP002341924 US AMERICAN CHEMICAL SOCIETY. WASHINGTON.
- CHEN Y. ET AL: 'Molecular cloning, tissue distribution and chromosomal localization of a novel member of the opioid receptor gene family' FEBS LETTERS vol. 347, 1994, pages 279 - 283, XP002978697
- SALVADORI ET AL BIOCHEMICAL AND BIOPHYSICAL RESEARCH COMMUNICATIONS. vol. 223, 1997, ACADEMIC PRESS INC. ORLANDO, FL., pages 640 - 643
- TANCREDI T. ET AL CHEMISTRY - A EUROPEAN JOURNAL. vol. 11, 2005, WILEY - V C H VERLAG GMBH & CO. KGAA, pages 2061 - 2070
- HAUPTMANN; WIDHOLM PLANT PHYSIOLOGY. vol. 70, 1982, AMERICAN SOCIETY OF PLANT PHYSIOLOGISTS, ROCKVILLE, MD., pages 30 - 34
- BÉDARD ET AL JOURNAL OF BACTERIOLOGY vol. 141, no. 1, 1980, AMERICAN SOCIETY FOR MICROBIOLOGY, pages 100 - 105
- WILSON ET AL BIOCHEMISTRY vol. 36, no. 15, 1997, AMERICAN CHEMICAL SOCIETY, pages 4542 - 4551
- GOLOVINSKY EVGENY: 'Biochemie der Antimetabolite', 2002, PROF. MARIN-DRINOV VERLAG DER BULGARISCHEN AKADEMIE DER WISSENSCHAFTEN, SOFIA (BG)

## Description

### FIELD OF THE INVENTION

The present invention relates to peptidomimetic opioid receptor-like 1 ("ORL-1") receptor ligands containing certain conformationally constrained amino acids that favor alpha-helix conformations. The ORL-1 receptor ligands of the present invention have one or more residues in an "address" segment to promote alpha-helix formation. The ligands of the present invention include ORL-1 receptor agonists that may be used as analgesics to treat pain in a patient in need of such treatment, and also may be used to treat anxiety, addiction, withdrawal and drug tolerance, as well as to enhance memory and learning.

### BACKGROUND OF THE INVENTION

The opioid system modulates several physiological processes including analgesia, stress responses, immune responses, respiration, and neuroendocrine function (Herz, Opioids 1993, Vol. 1, Springer-Verlag, Berlin). Pharmacological and molecular cloning studies have identified four opioid receptor types (µ, δ, κ, and ORL-1) that mediate these diverse effects (Miotto et al., The Pharmacology of Opioid Peptides (Ed. L. Tseng) 1995, 57-71, Harwood Acad. Publishers; Kieffer et al., Cell Mol. Neurobiol. 1995, 15:615-35). The opioid receptors are known to couple with pertussis toxin sensitive G proteins to modulate adenylyl cyclase activity and potassium and calcium channel currents (Handbook of Experimental Pharmacology, Vol.104/I:Opioids I (Herz, A; Ed.) 1993, Springer-Verlag, Berlin; Duggan and North, Pharm. Rev. 1983, 35:219-282).

Most clinically used opiates are µ-receptor ligands. For example, endorphins and enkephalins are endogenous ligands for the µ-receptor. Dynorphin A also has high affinity for µ-receptors, but has a higher affinity for κ-receptors. Morphine and other morphine-like agonists produce analgesia primarily through interaction with µ-receptors. Other physiological effects that are associated with µ-receptor activation include, but are not limited to, respiratory depression, miosis, reduced gastrointestinal motility, and euphoria (Pasternak, Clin. Neuropharmacol 1993, 16:1-18). As a result of the euphoria effect of µ-receptor ligands, many of these compounds are abused. *In situ* hybridization studies have shown that µ-receptor mRNA is present in brain regions associated with pain perception (*e.g.*, periaqueductal gray, spinal trigeminal nucleus, cunate and gracile nuclei, and thalamus), respiration (*e.g.*, nucleus of the solitary tract, nucleus ambiguus, and parabrachial nucleus), and nausea and vomiting (*e.g.*, neurons of the area postrema) (The Pharmacological Basis of Therapeutics, 9th edition (Eds Hardman, JG and Limbird, LE) 1996, McGraw-Hill, New York). It is hypothesized that addiction to certain analgesics occurs through hyperactivation of µ-receptors.

The ORL-1 receptor is a G-protein coupled receptor recently identified as a member of the opioid receptor family that includes the (µ-, δ-, and κ-opioid receptors (Chen et al., FEBS Lett. 1994, 347: 279-83; Keith et al, Regul. Pept. 1994, 54: 143-44; Wick et al., Mol. Brain Res. 1994, 27:37-44; Wang et al., FEBS Lett. 1994, 348:75-79; Mollereau et al, FEBS Lett. 1994, 341:33-38; Lachowicz et al., J. Neurochem. 1995, 64: 34-40; Bunzow et al., FEBS Lett. 1994, 347:284-88; and Fukuda et al, FEBS Lett. 1994, 343:42-46). A number of studies have demonstrated a broad spectrum of physiological functions of the ORL-1 receptor in both the central and peripheral nervous systems and in non-neuronal tissues. These functions include modulation of nociception (Meunier et al., Nature 1995, 377:532-5; Reinscheid et al., Science 1995, 270:792-94; Tian et a., Br. J. Pharmacology 1998, 124:21-6; Yamada et al., Br. J. Pharmacology 2002, 135:323-332; Yamada et al., J Med Chem 2000, 43:4667), locomotor activity (Reinscheid *et al., supra*), reversal of stress-induced analgesia (Mogil et al., Neuroscience 1996, 75:333-37), attenuation of stress responses (Jenck et al., PNAS USA 1997, 94:14854-58), modulation of learning and memory (Mamiya et al., Brain Res. 1998, 783:236-40; Manabe et al., Nature 1998, 394:577-81; and Sandin et al., Eur. J. Neurosci. 1997, 9:194-97), regulation of neurotransmitter and hormone release (Bryant et al., Brain Res. 1998, 807:228-33; Murphy et al., Neuroscience 1996, 75:1-4), modulation of kidney function (Kapusta et al., Life Sci. 1997, 60:PL15-21), and a potential role in neuronal differentiation (Buzas et al., J. Neuroschem. 1999, 72:1882-89; Saito et al, Biochem. Biophs. Res. Commun. 1995, 217:539-45; and Saito et al., J. Bio. Chem. 1996, 271:15615-22).

The endogenous agonist of this receptor is the heptadecapeptide, nociceptin (designated herein as "NC"), a 17 amino acid peptide having the sequence FGGFTGARKSARKLANQ (SEQ ID NO: 1) (Meunier *et al.,* 1995, *supra*), or orphanin FQ (Reinscheid *et al.,* 1995, *supra*). It has been suggested that the N-terminal portion of NC, *i.e.,* FGGF (SEQ ID NO: 2), which is often referred to as the "message", is primarily responsible for triggering the stimulation of the ORL-1 receptor. The remainder of NC, *i.e.,* TGARKSARKLANQ (SEQ ID NO: 3), is referred to as the "address", and is thought to be involved in binding and receptor specificity (Guerrini et al., J. Med. Chem. 1997, 40 (12): 1789-93).

The ORL-1 receptor has seven membrane-spanning domains. Due to its complex membrane-bound structure, the structure of the native NC-ORL-1 ligand-receptor complex cannot be resolved by conventional methods, such as X-ray crystallography or NMR spectroscopy. As a result, researchers have resorted to indirect methods of determining the three-dimensional structure of the ligand-receptor complex, such as molecular modeling (Huang et al., Acta Pharmacol. Sin. 2000, 21:536-46; and Facchiano et al., Protein Eng. 1999, 10:893-99), elucidation of the NC solution conformation by NMR (Biochem. Biophys. Res. Commun. 1997, 233:640-43), and synthesis of certain conformationally constrained NC analogs (Ambo et al., J. Med. Chem. 2001, 44:4015-18.

The rationale for the latter approach is based on the premise that conformational degrees of freedom in the native, linear, NC sequence might be significantly reduced by the introduction of conformational constraints. Such constraints can reduce the peptide's flexibility to a small number of predictable and/or experimentally solvable conformations. The experimentally determined affinities and/or efficacies of such constrained peptides, when taken together with the three-dimensional conformation imposed, may provide tangible clues regarding the bioactive structure of the peptide ligand.

Ambo *et al. (supra)* describes synthesis of a series of cyclic peptides by forming a side chain to side chain disulfide bond between two cysteine residues. Since NC does not contain cysteine in the native sequence, this article reported altering the native sequence by replacement by and/or insertion of a cysteine at various positions. In addition, the article described truncating the native sequence by eliminating certain residues from the C-terminus. The data presented in the article demonstrated that cyclizations at the N-terminal or middle portion of the sequence significantly diminish binding and functional potency at the ORL-1 receptor, while cyclization at the C-terminus yields agonists with potencies comparable to native NC. Thus, the bioactive form of NC is not likely cyclic or pseudo-cyclic at the N-terminus or in the middle portion of the native sequence. However, the Ambo *et al.* study reported making substantial alterations to the native NC sequence, which limits the ability to interpret the actual structure of the native ligand-receptor complex.

There is a continuing need in the art to develop ligands that are highly selective for one opioid receptor versus another. An understanding of how native NC binds to and activates the ORL-1 receptor at the molecular level would be a valuable tool that could be used to design molecules with high ORL-1 affinity and specificity. Such selective ligands may represent novel drugs for the treatment of pain, anxiety, cough and addiction, among other conditions, that minimize adverse effects due to interaction with other opioid receptors. The present invention addresses this and other needs in the art.

### SUMMARY OF THE INVENTION

The present invention provides an ORL-1 receptor ligand according to any one of claims 1 or 2, comprising a first portion which is, or which mimics, an N-terminal message segment from the native NC ligand; and a second portion, covalently attached to the first portion, which second portion is, or mimics, a C-terminal address segment from a native NC ligand, but which has been modified by the substitution of one or more amino acid residues with one or more amino acids or amino acid analogs that promote the formation of an alpha-helix, wherein the at least one amino acid analog is AIB. The native NC ligand preferably has the sequence of SEQ ID NO:I. The ORL-1 receptor ligand may be an ORL-1 receptor agonist, or, alternatively, an antagonist. In a particular embodiment, the ORL-1 receptor ligand of the present invention is an ORL-1 receptor agonist. In a further embodiment, the ORL-1 receptor ligand is a peptidomimetic having one or more amino acid analogs in the address segment that promote alpha helix formation, wherein the at least one amino acid analog is AIB. The second portion has an N-terminus at which it is covalently attached to a C-terminus of the first portion.

Other amino acid analogs (not claimed) that may be used as alpha-helix promoters to produce ORL-1 receptor ligands include indanes and backbone cyclizations of amino acids; biphenyls (Jacoby, Bioorg. Med. Chem. Lett. 2002, 12:891-893); and certain small molecule initiators (Meara et al., Proc. Eur. Pept. Symp. 1995, Meeting Date 1994, 692-693). Other alpha helix promoters (not claimed) are described in Horwell et al., Bioorg. Med. Chem. 1996, 4: 33-42; and in U.S. Patent Nos. 5,446,128 and 5,710,245. Regarding naturally occurring amino acids, comparative values of alpha-helix promoting properties are known in the art, and some amino acids, including leucine, have been found to have a pronounced helix-forming tendency (see, e.g., Cantor and Schimmel, Biophysical Chemistry, parts I and III, W.H. Freeman and Co., San Francisco, 1980, chapters 5 and 20).

The ORL-1 receptor ligands of the present invention may also be produced by combining different types of alpha-helix promoters into the same peptidomimetic.

In addition, the address segment of the ORL-1 receptor ligand of the present invention optionally includes a moiety substituted in place of the native carboxyl group at the C-terminus, which moiety is selected from any moiety that is neutral or positively charged at physiological pH, *e.g.,* an ester, ketone, or substituted or unsubstituted amide. In one example, the moiety is an amide group substituted in place of the native carboxyl group at the C-terminus. In another example, the carbonyl group at the C-terminus may be reduced to a methylene, thereby creating an ether or amine where the parent group was an ester or amide, respectively.

Alpha-helix promoters (X_{α}) include AIB. In one embodiment, the alpha-helix promoter (X_{α}) is AIB.

The present invention further provides an ORL-1 receptor ligand according to claim 1 as described above, wherein the address segment comprises a sequence selected from the group consisting of:
(X_{α})-RKS-A-RKL-A-NQ (SEQ ID NO:4);
A-RKS-(X_{α})-RKL-A-NQ (SEQ ID NO:5);
A-RKS-A-RKL-(X_{α})-NQ (SEQ ID NO:6);
(X_{α})-RKS-(X_{α})-RKL-A-NQ (SEQ ID NO:7);
(X_{α})-RKS-A-RKL-(X_{α})-NQ (SEQ ID NO:8);
A-RKS-(X_{α})-RKL-(X_{α})-NQ (SEQ ID NO:9);
(X_{α})-RKS-(X_{α})-RKL-(X_{α})-NQ (SEQ ID NO:10);
and the pharmaceutically acceptable salts and solvates thereof.

In one embodiment, each alpha-helix promoter (X_{α}) in any of SEQ ID NOS:4-10 is independently selected from AIB. In a preferred embodiment, each alpha-helix promoter (X_{α}) in SEQ ID NOS:4-10 is AIB. It is understood that the peptidomimetics of SEQ ID NOS:4-10, and all of those described herein below, are represented by the generally accepted single letter amino acid abbreviations.

Each ligand comprising the sequences of any of SEQ ID NOS:4-10 described above will preferably, but not necessarily, further comprise amino acids T and G, or analogs for either or both of T and G, or amino acid substitutions for either or both of T and G, at the N-terminus of the address segment.

The present invention further contemplates that either or both of the last two amino acid residues at the C-terminus of the address segment of native NC, which residues are N and Q, can be replaced by corresponding analogs for those amino acid residues, substituted with another amino acid, or deleted.

Each address segment further comprises a message segment attached at its N-terminus to form a complete peptidomimetic ORL-1 ligand. The message segment is the amino acid sequence FGGF (SEQ ID NO:2).

Thus, the present invention also provides an ORL-1 receptor ligand according to claim 3 as described above, which comprises a sequence selected from the group consisting of:
FGGF-TG-(X_{α})-RKS-A-RKL-A-NQ (SEQ ID NO:18);
FGGF-TG-A-RKS-(X_{α})-RKL-A-NQ (SEQ ID NO:19);
FGGF-TG-A-RKS-A-RKL-(X_{α})-NQ (SEQ ID NO:20);
FGGF-TG-(X_{α})-RKS-(X_{α})-RKL-A-NQ (SEQ ID NO:21);
FGGF-TG-(X_{α})-RKS-A-RKL-(X_{α})-NQ (SEQ ID NO:22;
FGGF-TG-A-RKS-(X_{α})-RKL-(X_{α})-NQ (SEQ ID NO:23);
FGGF-TG-(X_{α})-RKS-(X_{α})-RKL-(X_{α})-NQ (SEQ ID NO:24);
and the pharmaceutically acceptable salts and solvates thereof.

Each alpha-helix promoter (X_{α}) in any of SEQ ID NOS:18-24 is as described above for SEQ ID NOS: 4-10.

Although the native, human NC ligand is 17 amino acid residues in length, an NC peptide missing one or more of the four C-terminal amino acids (*i.e.*, LANQ) can retain activity at the ORL-1 receptor. Thus, the address segment can be shortened in length at the C-terminus and still retain activity. Accordingly, the present invention further contemplates peptidomimetics having any of the sequences described herein, but in which any one, two, or three, or all four, of the four native C-terminal amino acid residues are missing. In addition to the possibility of deletion, any one, two or three, or all four of the native C-terminal amino acid residues can also be substituted with another amino acid residue or amino acid analog, or some combination thereof. In a preferred embodiment, one or more of the four C-terminal amino acid residues may be substituted with a basic amino acid such as Arg, Lys, or His, preferably Arg or Lys, or an analog thereof.

In one embodiment, the ligand is a peptidomimetic of any of SEQ ID NOS: 18-24, but where any one, two, three or all four of the four C-terminal amino acid residues are missing or replaced by one or more other amino acid residues or amino acid analogs. For example, the present invention provides such an ORL-1 receptor ligand according to claim 2 corresponding to SEQ ID NOS: 21, 18, and 19, respectively, where one, two, three, or all of the four C-terminal amino acids are missing, comprising one of the following address sequences:
A-RKS-(X_{α})-RK (SEQ ID NO:48)
(X_{α})-RKS-A-RK (SEQ ID NO:49)
(X_{α})-RKS-(X_{α})-RK (SEQ ID NO:50)

In one embodiment, each alpha-helix promoter (X_{α}) in any of SEQ ID NOS:48-50 is as described above for SEQ ID NOS: 4-10. Each address segment further comprises a message segment attached at its N-terminus to form a complete peptidomimetic ORL-1 ligand. The message segment is the amino acid sequence FGGF (SEQ ID NO:2).

Accordingly, the present invention provides for ORL-1 ligands according to claim 4 comprising any one of the following sequences:
FGGF-TG-A-RKS-(X_{α})-RK (SEQ ID NO:51)
FGGF-TG-(X_{α})-RKS-A-RK (SEQ ID NO:52)
FGGF-TG-(X_{α})-RKS-(X_{α})-RK (SEQ ID NO:53)

In one embodiment, each alpha-helix promoter (X_{α}) in any of SEQ ID NOS:51-53 is as described above for SEQ ID NOS: 4-10.

In addition, any of the peptidomimetics of the present invention may have additional amino acid residues and/or amino acid analogs covalently attached at the C-terminus of the address segment. Accordingly, the present invention contemplates peptidomimetics ranging in length from 13 to 35 subunits, or from 13 to 30 subunits, or from 13 to 25 subunits, or from 13 to 20 subunits, and preferably from 13 to 17 subunits, wherein the subunits constitute the amino acid residues and amino acid analogs constituting the particular peptidomimetic.

The invention further provides an ORL-1 receptor ligand as described above, wherein the address segment comprises any sequence described herein above, but where the carboxyl group on the C-terminal amino acid residue, which in some cases is Q, is substituted with a moiety selected from any moiety that is neutral or positively charged at physiological pH, such as *e.g.,* an ester, ketone, or substituted or unsubstituted amide.

The present invention further provides an ORL-1 receptor ligand according to any one of claims 1 or 2 comprising:
(a) a first portion which is, an N-terminal message segment from a native NC ligand; and
(b) a second portion, covalently attached to the first portion, which second portion is, or mimics, a C-terminal address segment from the native NC ligand, but which further comprises means for promoting a specific secondary structural conformation selected from the group consisting of an alpha-helix.

The secondary structural conformation is an alpha-helix. Means for promoting alpha-helices include all those described herein.

The ligands of the present invention may be either ORL-1 receptor agonists or antagonists. Agonists and antagonists of the present invention can be distinguished from each other using standard functional assays known in the art, such as the assay described below in the Example section.

The ORL-1 ligands of the invention can be used, for example, in the manufacture of a medicament for treating pain in a patient in need of such treatment comprising administering to the patient an analgesic effective amount of the ligand. The ligands of the present invention can also be used in the manufacture of a medicament for treating anxiety, drug addiction, drug withdrawal or drug tolerance in a patient in need of such treatment, comprising administering to the patient a therapeutically effective amount of the ligand. Agonists of the present invention can also be used in the manufacture of a medicament for enhancing cognitive function, such as memory or learning, or to treat Alzheimer's Disease, in a patient in need of such treatment, comprising administering to the patient a therapeutically effective amount of the agonist. In each case, the "therapeutically effective amount" will depend, among other factors, on the condition being treated.

The present invention further provides the use in the manufacture of a medicament for modulating a pharmacological response from an ORL-1 receptor comprising contacting the ORL-1 receptor with a peptidomimetic of the present invention. The ORL-1 receptor modulated by this use may be present in a patient to be treated, or in a tissue sample, or in an *in vitro* cell culture or membrane preparation. In this regard, an ORL-1 receptor includes a full-length receptor, or a portion thereof such as, *e.g.,* a truncated version of the receptor, which portion comprises a ligand-binding portion of the receptor but which may lack part of the extracellular or cytoplasmic domain, especially at the C-terminus. The term "modulate" as used herein with respect to the ORL-1 receptor means the (i) inhibiting or activating the receptor, or (ii) directly or indirectly affecting the normal regulation of the receptor activity. Compounds that modulate ORL-1 receptor activity include full and partial agonists, full and partial antagonists, and inverse agonists (collectively referred to herein as "ORL-1 receptor ligands").

The present invention further provides the use of any of the ORL-1 agonists of the present invention in the manufacture of a medicament for treating pain, anxiety, drug addiction, drug withdrawal and/or drug tolerance, or for enhancing cognitive function, such as memory or learning, or for treating Alzheimer's Disease.

The present invention further provides the use of any of the ORL-1 receptor antagonists of the present invention in the manufacture of a medicament for treating pain, drug addiction, drug withdrawal or drug tolerance.

The present invention further provides pharmaceutical compositions comprising an ORL-1 receptor ligand of the present invention combined with a pharmaceutically acceptable carrier. In one embodiment, the ligand is an ORL-1 receptor agonist. In another embodiment, the ORL-1 receptor ligand is an ORL-1 receptor antagonist. In a preferred embodiment, the ORL-1 agonist or antagonist is present in the pharmaceutical composition in a concentration such that treatment of the particular condition will be effected when the appropriate dosage or treatment regimen is administered to the particular patient.

The present invention further provides a kit, comprising at least one sterile container comprising an ORL-1 receptor ligand, or pharmaceutical composition, of the present invention. In one embodiment, the ligand is an ORL-1 receptor agonist. In another embodiment, the ORL-1 receptor ligand is an ORL-1 receptor antagonist. The kit optionally further comprises a second container comprising a sterile, pharmaceutically acceptable diluent useful to dilute or dissolve the active ingredient in the first container. Where the ORL-1 receptor ligand is an ORL-1 receptor agonist, the kit optionally further comprises a printed label and/or a set of printed instructions directing the use of the agonist or pharmaceutical composition to treat pain, anxiety, drug addiction, drug withdrawal and/or drug tolerance, or to enhance cognitive function, in a patient in need of such treatment. Where the ORL-1 receptor ligand is an ORL-1 receptor antagonist, the kit optionally further comprises a printed label and/or a set of printed instructions directing the use of the agonist or pharmaceutical composition to treat pain, drug addiction, drug withdrawal and/or drug tolerance in a patient in need of such treatment.

The present invention further provides a method of screening for compounds capable of interacting with an ORL-1 receptor, comprising:
(a) obtaining a compound according to claim 1 and
(b) testing the compound for its ability to bind to an ORL-1 receptor, or for its ability to agonize or antagonize the ORL-1 receptor;
   wherein a compound determined to bind to, agonize, or antagonize, the ORL-1 receptor is identified as a compound capable of interacting with the ORL-1 receptor.

In one embodiment, the ORL-1 receptor is a human ORL-1 receptor.

The compound further comprises an N-terminal message segment from the native NC which is covalently attached to the address segment.

In another embodiment, the native nociceptin (NC) has the amino acid sequence of SEQ ID NO:1.

The one or more substitutions in the address segment serve to promote alpha-helix formation of the resulting compound. In a preferred embodiment, such substitutions are those described herein above.

The compound tested according to this method may further include modifications at the C-terminus, as described above.

The ability of the compound to bind to the ORL-1 receptor can be ascertained in a standard binding assay to measure its affinity (*e.g.,* Ki) for the receptor. The ability of the compound to agonize or antagonize the ORL-1 receptor (*e.g.*, EC₅₀ and Eₘₐₓ) can be ascertained using a standard functional assay to determine functional potency.

The present invention further provides a method of screening for compounds capable of modulating ORL-1 receptor activity, comprising:
(a) obtaining an ORL-1 ligand according to claim 1;
(b) combining the ligand of step (a) with an ORL-1 receptor, or a ligand-binding portion thereof, under conditions that permit or induce the formation of a ligand-receptor complex;
(c) exposing the ligand-receptor complex of step (b) to a test compound; and
(d) detecting: (i) displacement of the ligand from the ligand-receptor complex, or (ii) a change in ORL-1 receptor activity in the presence of the test agent;
wherein a compound that is determined to displace the ORL-1 ligand from the ligand-receptor complex, or to cause a change in ORL-1 receptor activity, is identified as a compound capable of modulating ORL-1 receptor activity.

In one embodiment, this method can be carried out using any standard format for a competition binding assay.

The ORL-1 receptor useful in either of the above-described methods is a mammalian ORL-1 receptor, or a portion thereof sufficient to test binding of the compound, or to test the ability of the compound to agonize or antagonize the ORL-1 receptor. In a preferred embodiment, the ORL-1 receptor is a primate ORL-1 receptor, and more preferably a human ORL-1 receptor.

Either of the above-described methods may alternatively be used to characterize the binding characteristics of a compound that has previously been identified as a compound capable of interacting with, or modulating the activity of, an ORL-1 receptor.

Any of the screening methods of the present invention may be conducted in any convenient format known in the art, such as, *e.g.,* in a 96-well plate format. The compound identified by any of the methods will typically be subjected to further analysis to determine whether, and/or to what degree, it can modulate activity of the ORL-1 receptor, as well as to determine the type of modulation. For example, the compound can be further analyzed to determine whether it functions as an agonist or antagonist at the ORL-1 receptor, and to determine its binding affinity and potency. In a preferred embodiment, the peptidomimetic ligand is detectably labeled, *e.g.,* with a radioisotope, to facilitate detecting, and quantifying the amount of, displacement of the peptidomimetic ligand from the ligand-receptor complex. These methods are useful in high throughput screening (HTS) to identify compounds capable of modulating the activity of the ORL-1 receptor. The methods are also useful to characterize the binding affinity or binding selectivity of a compound that has already been identified as a compound capable of modulating the activity of the ORL-1 receptor. The compound tested may be a small organic molecule, peptide, peptide mimetic, antibody or antibody fragment (such as, e.g, F(ab)₂, Fab', Fab, Fv fragments, and single chain antibodies). As used herein, the term "small organic molecule" is intended to refer to a molecule having a molecular weight of about 600 amu or less.

### BRIEF DESCRIPTION OF THE DRAWINGS

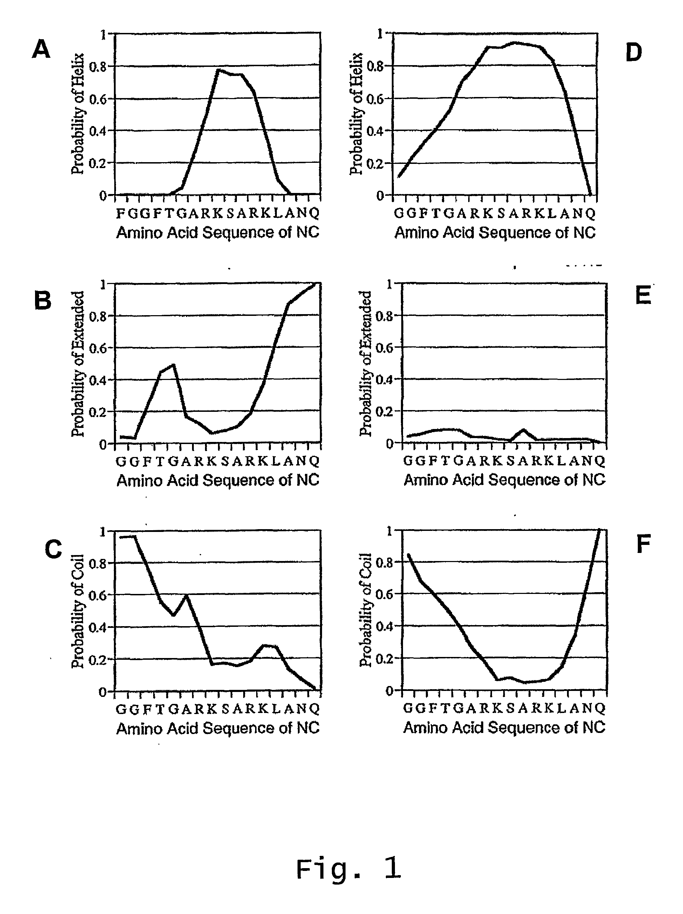

**FIGURE 1A-1F****.** The output of the GOR IV secondary structure prediction program is presented in Figs. 1A-1C, wherein (A) shows the relative probability of helix formation, (B) shows the relative probability of extended sheet formation, and (C) shows the relative probability of coil formation. The output of the PHD secondary structure prediction program is shown in Figs. 1D-1F, wherein (D) shows the relative probability of helix formation, (E) shows the relative probability of extended sheet formation, and (F) shows the relative probability of coil formation.

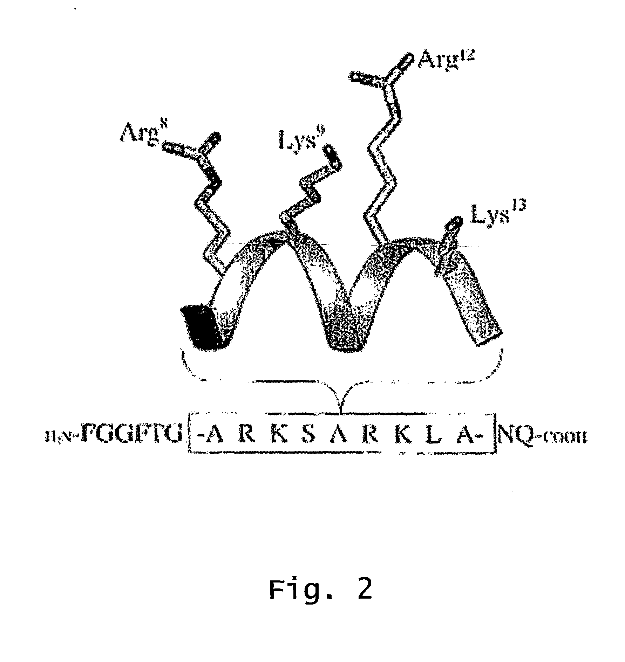

**FIGURE 2** is a diagram of the secondary structure of nociceptin (NC).

### DETAILED DESCRIPTION OF THE INVENTION

The present invention is based on the observation that the address segment of NC, *i.e.,* TGARKSARKLANQ (SEQ ID NO: 3), contains three regularly spaced alanine (Ala or A) residues at positions 3, 7, and 11, respectively, that are arranged in the ordered pattern: ARKxARKxA (SEQ ID NO: 46). This symmetrical repeat of Ala residues, separated by a regular repeat of the highly basic dipeptide Arg-Lys (RK), may represent the possibility of an amphipathic helix within this segment of the peptide. This concept is supported by the secondary structure predictions obtained when the primary sequence of NC was input into either a hierarchical neural network (such as PHD) or an information theory-based prediction algorithm (such as GOR IV). For the former, the program PHD (Rost and Sander, J. Mol. Biol. 1993, 232:584-99) predicts that NC has a high probability of adopting a helical conformation spanning the sequence from Gly-6 to Leu-14. For the latter, the GOR IV algorithm (Garnier et al., Meth. Enzymol. 1996, 266:540-53) predicts that NC has a high probability of adopting a helical conformation spanning Arg-8 to Lys-13. The program prediction outputs from each of these programs are presented in Figures 1A-1F.

To more fully explore this structural hypothesis, a series of full-length NC analogs were prepared which contain either N-methyl alanine (NMA) or α-methyl alanine (AIB) as replacements for the alanine at position 7, 11 or 15 of NC (SEQ ID NO:1). N-methyl substitution on an amino acid in a given peptide sequence is known to disfavor the adoption of local φ, ψ angles that would correspond to a helical secondary structure (φ, ψ. approximately -60°, -60°), while favoring an extended backbone (φ,ψ approximately 180°, 180°). The complimentary, although contrasting, C^{α} methyl modification has been shown to favor a helical conformation, rather than an extended one (Topics on Current Physics, Metzyer, R.H. (ed.), Springer Verlag, New York 1981, 26:41-79; Momany and Chuman, Meth. Enzymol. 1986, 124:3-17; and Chakravarty et al., J. Med. Chem. 1993, 36:2569-71). These conformational preferences apply only to the backbone φ, ψ angles (wherein φᵢ, ψᵢ correspond to backbone dihedral angles for residue i defined by the four adjacent amino acid backbone atoms Cᵢ₋₁-Nᵢ-C^{α}ᵢ-Cᵢ and Nᵢ-C^{α}ᵢ-Cᵢ-Nᵢ₊₁, respectively) of the same amino acid bearing the additional methyl group. If the bioactive form of NC adopts an amphipathic helix within the address segment of the sequence, the AIB-containing peptidomimetics would have binding affinities and potencies similar or superior to NC, while the NMA-containing peptidomimetics should lose affinity and be less potent.

Thus, several full-length NC analogs were prepared that consist of an N-terminal message segment from NC, and a C-terminal address segment, wherein the address segment includes one or more amino acid analogs that promote formation of either an alpha-helix or an extended β-strand. More specifically, several NC analogs were prepared that contain an address segment comprising one or more residues of α-methyl alanine (AIB), and several NC analogs were prepared that contain an address segment comprising one or more residues of N-methyl alanine (NMA). In addition, several such NC analogs were prepared that also include an amide group in place of the native carboxyl group at the C-terminus.

### Definitions

The following defined terms are used throughout the present specification, and should be helpful in understanding the scope and practice of the present invention.

The term "peptide" is used in its broadest sense to refer to a compound comprising two or more subunit amino acids linked together by peptide bonds.

The term "amino acid" refers to any of the twenty naturally occurring L-amino acids.

The term "amino acid analog" refers to an amino acid that is other than any of the twenty naturally occurring L-amino acids, such as, *e.g.,* a D-amino acid; or that is a non-naturally occurring amino acid that could be synthetically prepared by modifying a naturally occurring or non-naturally occurring amino acid; or that is any naturally occurring or non-naturally occurring chemical derivative of a naturally occurring amino acid; or that is any chemical compound that is recognized in the art of peptide chemistry to be a derivative or chemically modified form of an amino acid. Examples of amino acid analogs include AIB and NMA and various "designer" amino acids (*e.g.,* α-methyl amino acids, C^{α}-methyl amino acids, and N^{α}-methyl amino acids) designed to confer special properties, such as conformational constraints, on peptidomimetics of the invention. Other examples of amino acid analogs are described below.

The NC analogs described above are peptidomimetics. The term "peptidomimetic" refers to a compound comprising: (i) a combination of at least one amino acid and at least one amino acid analog covalently linked together in linear fashion; or (b) a combination of at least two amino acid analogs covalently linked together in linear fashion; or (c) a combination of two or more amino acids and/or amino acid analogs covalently linked together in linear fashion by a bond that is other than a peptide bond, such as, *e.g.,* an ester or ether linkage. For example, peptidomimetics of the present invention may comprise D-amino acids, or "designer" amino acid analogs, or combinations of L-amino acids with D-amino acid and/or "designer" amino acid analogs (*e.g.*, α-methyl amino acids C^{α}**-**methyl amino acids, and N^{α}-methyl amino acids), among other possible combinations not claimed.

The following non-naturally occurring amino acid analogs may be incorporated into peptidomimetics not claimed to introduce particular conformational motifs: 1,2,3,4-tetrahydroisoquinoline 3-carboxylate (Kazmierski et al., J. Am. Chem. Soc. 1991, 113:2275-2283); (2S,3S)-methyl-phenylalanine, (2S,3R)-methyl-phenylalanine, (2R,3S)-methyl-phenylalanine and (2R,3R)-methyl-phenylalanine (Kazmierski and Hruby, Tetrahedron Lett. 1991); 2-aminotetrahydronaphthalene-2-carboxylic acid (Landis, Ph.D. Thesis, University of Arizona, 1989); hydroxy-1,2,3,4-tetrahydroisoquinoline-3-carboxylate (Miyake et al., J. Takeda Res. Labs. 1989, 43:53-76); β-carboline (D and L) (Kazmierski, Ph.D. Thesis, University of Arizona, 1988); and HIC (histidine isoquinoline carboxylic acid) (Zechel et al., Int. J. Pept. Protein Res. 1991, 38:131-8).

In addition, the following amino acid analogs may be incorporated to induce or favor specific secondary structures not claimed: β-sheet inducing analogs (Kemp et al., Tetrahedron Lett. 1988, 29:5081-5082); and α-helix inducing analogs (Kemp et al., Tetrahedron Lett. 1988, 29:4935-4938). Additional analogs are described in the following references: Nagai and Sato, Tetrahedron Lett. 1985, 26:647-650; DiMaio et al., J. Chem. Soc. Perkin Trans. 1989, p. 1687; also a Gly-Ala turn analog (Kahn et al., Tetrahedron Lett. 1989, 30:2317); amide bond isostere (Jones et al., Tetrahedron Lett. 1988, 29:3853-3856); tretrazol (Zabrocki et al., J. Am. Chem. Soc. 1988, 110:5875-5880); DTC (Samanen et al., Int. J. Protein Pep. Res. 1990, 35:501-509); and analogs taught in Olson et al., J. Am. Chem. Sci. 1990, 112:323-333 and Garvey et al., J. Org. Chem. 1990, 56:436.

The following specific NC analogs were prepared, and studied using a number of human ORL-1 receptor binding and functional assays:
FGGFTG-(AIB)-RKS-A-RKL-A-NQ (SEQ ID NO:32);
FGGFTG-A-RKS-(AIB)-RKL-A-NQ (SEQ ID NO:33);
FGGFTG-A-RKS-A-RKL-(AIB)-NQ (SEQ ID NO:34);
FGGFTG-(NMA)-RKS-A-RKL-A-NQ (SEQ ID NO:35);
FGGFTG-A-RKS-(NMA)-RKL-A-NQ (SEQ ID NO:36);
FGGFTG-A-RKS-A-RKL-(NMA)-NQ (SEQ ID NO:37);
FGGFTG-(AIB)-RKS-A-RKL-A-NQ-C(O)NH₂ (SEQ ID NO:38);
FGGFTG-A-RKS-(AIB)-RKL-A-NQ-C(O)NH₂ (SEQ ID NO:39);
FGGFTG-A-RKS-A-RKL-(AIB)-NQ-C(O)NH₂ (SEQ ID NO:40);
FGGFTG-(NMA)-RKS-A-RKL-A-NQ-C(O)NH₂ (SEQ ID NO:41);
FGGFTG-A-RKS-(NMA)-RKL-A-NQ-C(O)NH₂ (SEQ ID NO:42);
FGGFTG-A-RKS-A-RKL-(NMA)-NQ-C(O)NH₂ (SEQ ID NO:43); and
FGGFTG-(AIB)-RKS-(AIB)-RKL-A-NQ-C(O)NH₂ (SEQ ID NO:44).

The results obtained from these studies strongly suggest that the adoption of an amphipathic helix within the address segment of NC in the receptor-bound state is favored. Moreover, several of the peptidomimetics have binding affinities and potencies that are superior to the native NC ligand, making them the most potent ORL-1 receptor agonists identified.

The following specific NC analogs were also prepared:
FGGF-TG-A-RKS-(AIB)-RK-NH₂ (SEQ ID NO:54)
FGGF-TG-(AIB)-RKS-A-RK-NH₂ (SEQ ID NO:55)
FGGF-TG-(AIB)-RKS-(AIB)-RK-NH₂ (SEQ ID NO:56)

The binding characteristics of these peptides can be investigated using the human ORL-1 receptor binding and functional assays described in Example 1, or other assays known in the art.

As used herein, the term "ORL-1 receptor" refers to a mammalian ORL-1 receptor, such as an ORL-1 receptor from a primate or a companion animal. In a preferred embodiment, the ORL-1 receptor is a human ORL-1 receptor, which can be prepared for testing according to the procedures described below, or as described in the publications cited herein above. The ORL-1 receptor may be full length or a portion thereof such as, *e.g.,* a truncated version of the receptor, comprising a ligand-binding portion of the receptor. The ORL-1 receptor may be present in a patient being treated for a particular condition treatable by modulation of the ORL-1 receptor. Alternatively, the ORL-1 receptor, or portion thereof, may be present in an *in vitro* receptor assay preparation useful to test for, or characterize, ligand binding and/or receptor activation, such as that described below in the Example section.

Certain preferred embodiments of the present invention are described below. In so far as the description refers to certain components of the invention with approximations, *e.g.,* the terms "about" or "approximately", these terms shall generally mean an acceptable degree of error for the quantity measured given the nature or precision of the measurements. Typical, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 5-fold and more preferably within 2-fold of a given value. Numerical quantities given herein are approximate unless stated otherwise, meaning that the term "about" or "approximately" can be inferred when not expressly stated.

The peptides and peptidomimetics of the present invention can be prepared by any suitable method, *e.g.,* solid phase synthesis. The coupling of amino acids and amino acid analogs may be accomplished by techniques familiar to those in the art and provided, for example, in Stewart and Young, 1984, Solid Phase Synthesis, Second Edition, Pierce Chemical Co., Rockford, IL. Amino acids and amino acid analogs used for peptide or peptidomimetic synthesis may be standard Boc (N^{α}-amino-protected N^{α}-t-butyloxycarbonyl) amino acid or amino acid analog resin with the standard deprotecting, neutralization, coupling and wash protocols of the original solid phase procedure of Merrifield (J. Am. Chem. Soc. 1963, 85:2149-2154), or the base-labile N^{α}-amino protected 9-fluorenylmethoxycarbonyl (Fmoc) amino acids first described by Carpino and Han (J. Org. Chem. 1972, 37:3403-3409). Both Fmoc and Boc α-amino protected amino acids and amino acid analogs can be obtained from Fluka, Bachem, Advanced Chemtech, Sigma, Cambridge Research Biochemical, Bachem, or Peninsula Labs or other chemical companies familiar to those who practice this art. In addition, the method of the invention can be used with other Nα-protecting groups that are familiar to those skilled in this art. Many methods of activation may be used in the practice of the invention and include, for example, preformed symmetrical anhydrides (PSA), preformed mixed anhydride (PMA), acid chlorides, active esters, and *in situ* activation of the carboxylic acid, as described by Fields and Noble (Int. J. Pept. Protein Res. 1990, 35:161-214). Solid phase peptide synthesis may be accomplished by techniques familiar to those in the art and provided, for example, in Stewart and Young (Solid Phase Synthesis, Second Edition, Pierce Chemical Co., Rockford, IL, 1984; Fields and Noble, *supra),* or using automated synthesizers, such as sold by ABS.

The completeness of coupling should be assessed. Those skilled in the art would be familiar with the well known quantitative monitoring tests such as ninhydrin (the Kaiser test), picric acid, 2,4,6-trinitro-benzenesulfonic (TNBS), fluorescamine, and chloranil, which are based on reagent reaction with free amino groups to produce a chromophoric compound. If imino acids (*e.g.*, Pro and Hyp) are used, isatin monitoring is a preferred method (Fields and Noble, *supra*). Quantification of reaction completeness may be monitored during the course of the reaction, *e.g.,* as described by Salisbury *et al.* (International Patent Publication No. WO91/03485).

If the coupling reaction is incomplete as determined by this test, the reaction can be forced to completion by several methods familiar to those in the art, including (a) a second coupling using a one- to five-fold excess of protected amino acid or amino acid analog, (b) an additional coupling using different or additional solvents (*e.g.*, trifluoroethane), or (c) the addition of chaotropic salts, *e.g.,* NaClO₄ or LiBr (Klis and Stewart, "Peptides: Chemistry, Structure and Biology"; In: Rivier and Marshall, eds., ESCOM Publ., 1990, p. 904-906).

The present invention contemplates the use of any of the peptidomimetics described above or their pharmaceutically acceptable salts or solvates.

The phrase "pharmaceutically acceptable salt," as used herein, is a salt formed from an acid and, *e.g.,* a basic nitrogen group of a peptidomimetic of the present invention. Preferred salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate (*i.e.,* 1,1'-methylene-bis-(2-hydroxy-3-naphthoate)) salts. The term "pharmaceutically acceptable salt" also refers to a salt prepared from a peptidomimetic having an acidic functional group, such as a carboxylic acid functional group, and a pharmaceutically acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy substituted mono-, di-, or trialkylamines; dicyclohexylamine; tributyl amine; pyridine; N-methyl,N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N, N,-di-lower alkyl-N-(hydroxy lower alkyl) amines, such as N,N,-dimethyl-N-(2-hydroxyethyl)amine, or tri-(2-hydroxyethyl)amine; N-methyl-D-glucamine; and amino acids such as arginine, lysine, and the like.

Peptidomimetics of the present invention have asymmetric centers and therefore may exist in different enantiomeric and diastereomic forms. A peptidomimetic can be in the form of an optical isomer or a diastereomer. All of these forms, and their uses, are encompassed by the present invention.

In addition, one or more hydrogen, carbon or other atoms of a peptidomimetic can be replaced by an isotope of hydrogen, carbon or other atom. Such compounds are useful as research and diagnostic tools in metabolism studies, in pharmacokinetic studies, or in binding assays, such as those described herein, and are encompassed by the present invention.

The peptidomimetics of the present invention may be formulated into pharmaceutical compositions further comprising pharmaceutically acceptable carriers. Suitable pharmaceutically acceptable carriers include, but are not limited to, ethanol, water, glycerol, aloe vera gel, allantoin, glycerin, vitamin A and E oils, mineral oil, PPG2 myristyl propionate, vegetable oils and solketal.

The pharmaceutical composition further comprise other pharmaceutically acceptable additives, such as a flavorant, a sweetener, a preservative, a dye, a binder, a suspending agent, a dispersing agent, a colorant, a disintegrant, an excipient, a diluent, a lubricant, a plasticizer, an edible oil, an anti-oxidant, a pH stabilizer, or any combination of any of the foregoing.

Suitable binders include, but are not limited to, starch, gelatin, natural sugars, such as glucose, sucrose and lactose, corn sweeteners, natural and synthetic gums, such as acacia, tragacanth, vegetable gum, and sodium alginate, carboxymethylcellulose, polyethylene glycol, waxes, and the like. Suitable disintegrants include, but are not limited to, starch, such as corn starch, or methyl cellulose, agar, bentonite, xanthan gum and the like. Suitable lubricants include, but are not limited to, sodium oleate, sodium stearate, magnesium stearate, sodium acetate, and the like. The composition may also include suitable preservatives, *e.g.,* sodium benzoate, and other additives the may render the composition more suitable for ingestion and or injection, *e.g.,* sodium chloride, which affects the osmolarity of the preparation. A suitable suspending agent is, but is not limited to, bentoite. Suitable dispersing and suspending agents include, but are not limited to, synthetic and natural gums, such as vegetable gum, tragacanth, acacia, alginate, dextran, sodium carboxymethylcellulose, methylcellulose, polyvinylpyrrolidone and gelatin. Suitable edible oils include, but are not limited to, cottonseed oil, corn oil, palm oil, sesame oil, coconut oil and peanut oil. A suitable pharmaceutical diluent is, but is not limited to, water, saline, or lactated Ringer's solution. Examples of additional additives include, but are not limited to, sorbitol, talc, stearic acid, and dicalcium phosphate.

The peptidomimetics of the present invention can be further modified to improve certain beneficial properties such as, *e.g.,* to increase resistance of the molecule to metabolic degradation or to slow clearance of the molecule from the blood. For example, a peptidomimetic of the present invention can be conjugated to a molecule such as a long chain polyethylene glycol (PEG). Conjugation is preferably located at the C-terminus of the peptidomimetic, or at a sub-terminal amino acid residue or amino acid analog somewhere in the C-terminal portion of the molecule. Methods of "pegylating" peptides are described in the art, include in PCT International Publication WO 92/16221, which was published October 1, 1992.

Pharmaceutical compositions of the present invention may be formulated as unit dosage forms, such as tablets, pills, capsules, boluses, powders, granules, sterile parenteral solutions, sterile parenteral suspensions, sterile parenteral emulsions, elixirs, tinctures, metered aerosol or liquid sprays, drops, ampoules, autoinjector devices or suppositories. Unit dosage forms may be used for oral, parenteral, intranasal, sublingual or rectal administration, or for administration by inhalation or insufflation, transdermal patches, and a lyophilized composition.

Solid unit dosage forms may be prepared by mixing the peptides of the present invention with a pharmaceutically acceptable carrier and any other desired additives as described above. The mixture is typically mixed until a homogeneous mixture of the compound of the present invention and the carrier and any other desired additives are formed, *i.e.,* until the compound is dispersed evenly throughout the composition.

Tablets or pills can be coated or otherwise compounded to a unit dosage form, which has delayed and/or prolonged action, such as time release and sustained release unit dosage forms. For example, the tablet or pill can comprise an inner dosage and an outer dosage component, the latter being in the form of an envelope over the former. The two components can be separated by an enteric layer, which serves to resist disintegration in the stomach and permits the inner component to pass intact into the duodenum or to be delayed in release.

Biodegradable polymers for controlling the release of the compound, include, but are not limited to, polylactic acid, polyepsilon caprolactone, polyhydroxy butyric acid, polyorthoesters, polyacetals, polydihydropyrans, polyanhydrides, polycyanoacrylates, cross-linked or amphipathic block copolymers of hydrogels, cellulosic polymers, and polyacrylates.

Liquid unit dosage forms include, but are not limited to, aqueous solutions, suitably flavoured syrups, aqueous or oil suspensions, and flavored emulsions with edible oils, as well as elixirs and similar pharmaceutical vehicles. These dosage forms may be prepared by dissolving or suspending the compound of the present invention in the liquid carrier.

Topical preparations typically contain a suspending agent and optionally, an antifoaming agent. Such topical preparations may be liquid drenches, alcoholic solutions, topical cleansers, cleansing creams, skin gels, skin lotions, and shampoos in cream or gel formulations (including, but not limited to aqueous solutions and suspensions).

The pharmaceutical composition or unit dosage forms of the present invention may be administered by a variety of routes such as intraveneous, intratracheal, subcutaneous, oral, parenteral, buccal, sublingual, opthalmic, pulmonary, transmucosal, transdermal, and intramuscular. Unit dosage forms also can be administered in intranasal form via topical use of suitable intranasal vehicles, or via transdermal routes, using those forms of transdermal skin patches known to those of ordinary skill in the art.

Pharmaceutical compositions and unit dosage forms of the present invention for administration parenterally, and in particular by injection, typically include a pharmaceutically acceptable carrier, as described above. A preferred liquid carrier for depot forms is vegetable oil. Injection may be, for example, intravenous, epidural, intrathecal, intramuscular, intraruminal, intratracheal, or subcutaneous for purposes of depot delivery and sustained effect.

The compounds, pharmaceutical compositions, or unit dosage forms of the present invention also can be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds of the present invention also may be delivered by the use of monoclonal antibodies as individual carriers to which the peptides are coupled. The peptides of the present invention may also be coupled with soluble polymers as targetable drug carriers. Such polymers include, but are not limited to, polyvinyl pyrrolidone, pyran copolymer, polyhydroxypropylmethacryl-amidephenol, polyhydroxy-ethylaspartamide-phenol, and polyethyl-eneoxideopolylysine substituted with palmitoyl residues.

A transdermal dosage form also is contemplated by the present invention. Transdermal forms may be a diffusion-driven transdermal system (transdermal patch) using either a fluid reservoir or a drug-in-adhesive matrix system. Other transdermal dosage forms include, but are not limited to, topical gels, lotions, ointments, transmucosal systems and devices, and iontophoretic (electrical diffusion) delivery systems. Transdermal dosage forms may be used for timed release and sustained release of the compound of the present invention.

The pharmaceutical compositions or unit dosage forms of the present invention may be administered to an animal, preferably a human being, in need thereof to agonize or antagonize the activity of the ORL-1 receptor.

The peptidomimetic pharmaceutical composition of the present invention may be used to treat various conditions, such as pain, anxiety, addiction, drug withdrawal, or drug tolerance, or to enhance cognitive function, such as memory or learning. The peptidomimetic pharmaceutical composition or unit dosage form of the present invention may be administered alone at appropriate dosages defined by routine testing in order to obtain optimal interaction with the ORL-1 receptor while minimizing any potential toxicity.

The daily dosage of the compounds and compositions of the present invention may vary according to a variety of factors such as underlying disease states, the individual's condition, weight, sex and age and the mode of administration. For oral administration, the pharmaceutical compositions can be provided in the form of scored or unscored solid unit dosage forms containing 0.01, 0.05, 0.1, 0.5, 1.0, 2.5, 5.0, 10.0, 15.0, 25.0, or 50.0 milligrams of the compound of the present invention for the symptomatic adjustment of the dosage to the patient to be treated. An effective amount of the compound may be supplied at a dosage level of from about 0.01 mg/kg to about 100 mg/kg of body weight per day. In a preferred embodiment, the effective amount of the compound of the invention is administered by any appropriate method to achieve a blood concentration of from about 500 pg/ml to about 1500ng/ml, and preferably from about 100 ng/ml to about 1000 ng/ml.

In a preferred embodiment, an analgesic effective amount of the peptidomimetic is administered to the patient in need of analgesic treatment. An "analgesic effective amount" is that amount of the peptidomimetic that reduces or alleviates pain in the patient, as determined by a difference in the degree of pain suffered by the patient before, and during or after treatment, taking into account other factors such as age, weight, gender, and the general condition of the patient, as well as the route of administration of the compound, in view of the results of clinical studies.

A "therapeutically effective amount" is that amount of the peptidomimetic that can treat, reduce, ameliorate, palliate, or prevent a condition in a patient, such as anxiety, addiction, drug withdrawal, or drug tolerance.

Alternatively, a "therapeutically effective amount" is that amount of the peptidomimetic that can enhance, increase, stimulate, or prevent a reduction in cognitive function, such as memory or learning, in a patient.

A "receptor agonizing effective amount" of the peptidomimetic may be administered to a patient in need thereof. Such an amount is that amount of the peptidomimetic that binds to and stimulates receptor function. Conversely, a "receptor antagonizing effective amount" is that amount of the peptidomimetic that binds to and inhibits receptor function. Such amounts may be determined for the individual patient, taking into consideration such factors as age, weight, gender, and the general condition of the patient, as well as the route of administration of the compound, in view of the results of clinical studies.

The dosage regimen utilizing the peptides of the present invention is selected in accordance with a variety of factors including age, weight, gender, and the general condition of the patient; the severity of the condition to be treated; the route of administration; the renal and hepatic function of the patient; and the particular peptidomimetic employed. A physician or veterinarian of ordinary skill can readily determine and prescribe the effective amount of the compound required to treat the appropriate condition in a patient in need of such treatment. Optimal precision in achieving concentrations of drug within the range that yields efficacy without toxicity requires a regimen based on the kinetics of the drug's availability to target sites in view of the results of clinical studies. This involves a consideration of the absorption, distribution, metabolism, and excretion of a drug.

The pharmaceutical composition or unit dosage form may be administered in a single daily dose, or the total daily dosage may be administered in divided doses of two, three or four times daily, or as a continual infusion.

In addition, co-administration or sequential administration of other therapeutic agents may be desirable. For combination treatment with more than one therapeutic agent, where the active agents are in separate dosage formulations, the active agents can be administered concurrently, or at separately staggered times. The dosage amount may be adjusted when combined with other active agents as described above to achieve desired effects. Alternatively, unit dosage forms of these various active agents may be independently optimized and combined to achieve a synergistic result wherein the pathology or condition being treated is reduced (or enhanced) to a degree that would result in a greater than additive response.

The other therapeutic agent includes, but is not limited to, an opioid agonist; a non-opioid analgesic; a non-steroid antiinflammatory agent; a Cox-II inhibitor; an antiemetic; a β-adrenergic blocker; an anticonvulsant; an antidepressant; a Ca2+-channel blocker; an anticancer agent; an anti-anxiety agent; an agent for treating or preventing an addictive disorder; an agent for treating or preventing Parkinson's disease; and mixtures thereof.

Effective amounts of the other therapeutic agents are well known to those skilled in the art. However, it is well within the skilled artisan's purview to determine the other therapeutic agent's optimal effective-amount range. In one embodiment of the invention where another therapeutic agent is administered to a patient, the effective amount of a compound of the present invention is less than its effective amount where the other therapeutic agent is not administered.

Useful opioid agonists include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, pharmaceutically acceptable salts thereof, and mixtures thereof.

In certain preferred embodiments, the opioid agonist is selected from codeine, hydromorphone, hydrocodone, oxycodone, dihydrocodeine, dihydromorphine, morphine, tramadol, oxymorphone, pharmaceutically acceptable salts thereof, and mixtures thereof.

Useful non-opioid analgesics include non-steroidal anti-inflammatory agents, such as ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, and pharmaceutically acceptable salts thereof, and mixtures thereof. Other suitable non-opioid analgesics include the following, non-limiting, chemical classes of analgesic, antipyretic, nonsteroidal antiinflammatory drugs: salicylic acid derivatives, including aspirin, sodium salicylate, choline magnesium trisalicylate, salsalate, diflunisal, salicylsalicylic acid, sulfasalazine, and olsalazin; para-aminophenol derivatives including acetaminophen and phenacetin; indole and indene acetic acids, including indomethacin, sulindac, and etodolac; heteroaryl acetic acids, including tolmetin, diclofenac, and ketorolac; anthranilic acids (fenamates), including mefenamic acid, and meclofenamic acid; enolic acids, including oxicams (piroxicam, tenoxicam), and pyrazolidinediones (phenylbutazone, oxyphenthartazone); and alkanones, including nabumetone. For a more detailed description of the NSAIDs, see Paul A. Insel, Analgesic-Antipyretic and Antiinflammatory Agents and Drugs Employed in the treatment of Gout in Goodman & Gilman*s The Pharmacological Basis of Therapeutics, 617-57 (Perry B. Molinhoff and Raymond W. Ruddon, Eds., Ninth Edition, 1996), and Glen R. Hanson Analgesic, Antipyretic and Anti-Inflammatory Drugs in Remington: The Science and Practice of Pharmacy Vol II, 1196-1221 (A. R. Gennaro, Ed. 19th Ed. 1995). Suitable Cox-II inhibitors and 5-lipoxygenase inhibitors, as well as combinations thereof, are described in U.S. Patent No. 6,136,839. Cox-II inhibitors include, but are not limited to, rofecoxib, celecoxib, and valdecoxib.

Useful antimigraine agents include, but are not limited to, alpiropride, dihydroergotamine, dolasetron, ergocornine, ergocorninine, ergocryptine, ergot, ergotamine, flumedroxone acetate, fonazine, lisuride, lomerizine, methysergide oxetorone, pizotyline, and mixtures thereof.

The other therapeutic agent can also be an antiemetic agent. Useful antiemetic agents include, but are not limited to, metoclopromide, domperidone, prochlorperazine, promethazine, chlorpromazine, trimethobenzamide, ondansetron, granisetron, hydroxyzine, acetylleucine monoethanolamine, alizapride, azasetron, benzquinamide, bietanautine, bromopride, buclizine, clebopride, cyclizine, dimenhydrinate, diphenidol, dolasetron, meclizine, methallatal, metopimazine, nabilone, oxyperndyl, pipamazine, scopolamine, sulpiride, tetrahydrocannabinol, thiethylperazine, thioproperazine, tropisetron, and mixtures thereof.

Useful β-adrenergic blockers include, but are not limited to, acebutolol, alprenolol, amosulabol, arotinolol, atenolol, befunolol, betaxolol, bevantolol, bisoprolol, bopindolol, bucumolol, bufetolol, bufuralol, bunitrolol, bupranolol, butidrine hydrochloride, butofilolol, carazolol, carteolol, carvedilol, celiprolol, cetamolol, cloranolol, dilevalol, epanolol, esmolol, indenolol, labetalol, levobunolol, mepindolol, metipranolol, metoprolol, moprolol, nadolol, nadoxolol, nebivalol, nifenalol, nipradilol, oxprenolol, penbutolol, pindolol, practolol, pronethalol, propranolol, sotalol, sulfinalol, talinolol, tertatolol, tilisolol, timolol, toliprolol, and xibenolol.

Useful anticonvulsants include, but are not limited to, acetylpheneturide, albutoin, aloxidone, aminoglutethimide, 4-amino-3-hydroxybutyric acid, atrolactamide, beclamide, buramate, calcium bromide, carbamazepine, cinromide, clomethiazole, clonazepam, decimemide, diethadione, dimethadione, doxenitroin, eterobarb, ethadione, ethosuximide, ethotoin, felbamate, fluoresone, gabapentin, 5-hydroxytryptophan, lamotrigine, magnesium bromide, magnesium sulfate, mephenytoin, mephobarbital, metharbital, methetoin, methsuximide, 5-methyl-5-(3-phenanthryl)-hydantoin, 3-methyl-5-phenylhydantoin, narcobarbital, nimetazepam, nitrazepam, oxcarbazepine, paramethadione, phenacemide, phenetharbital, pheneturide, phenobarbital, phensuximide, phenylmethylbarbituric acid, phenytoin, phethenylate sodium, potassium bromide, pregabaline, primidone, progabide, sodium bromide, solanum, strontium bromide, suclofenide, sulthiame, tetrantoin, tiagabine, topiramate, trimethadione, valproic acid, valpromide, vigabatrin, and zonisamide.

Useful antidepressants include, but are not limited to, binedaline, caroxazone, citalopram, dimethazan, fencamine, indalpine, indeloxazine hydrocholoride, nefopam, nomifensine, oxitriptan, oxypertine, paroxetine, sertraline, thiazesim, trazodone, benmoxine, iproclozide, iproniazid, isocarboxazid, nialamide, octamoxin, phenelzine, cotinine, rolicyprine, rolipram, maprotiline, metralindole, mianserin, mirtazepine, adinazolam, amitriptyline, amitriptylinoxide, amoxapine, butriptyline, clomipramine, demexiptiline, desipramine, dibenzepin, dimetacrine, dothiepin, doxepin, fluacizine, imipramine, imipramine N-oxide, iprindole, lofepramine, melitracen, metapramine, nortriptyline, noxiptilin, opipramol, pizotyline, propizepine, protriptyline, quinupramine, tianeptine, trimipramine, adrafinil, benactyzine, bupropion, butacetin, dioxadrol, duloxetine, etoperidone, febarbamate, femoxetine, fenpentadiol, fluoxetine, fluvoxamine, hematoporphyrin, hypericin, levophacetoperane, medifoxamine, milnacipran, minaprine, moclobemide, nefazodone, oxaflozane, piberaline, prolintane, pyrisuccideanol, ritanserin, roxindole, rubidium chloride, sulpiride, tandospirone, thozalinone, tofenacin, toloxatone, tranylcypromine, L-tryptophan, venlafaxine, viloxazine, and zimeldine.

Useful Ca²⁺-channel blockers include, but are not limited to, bepridil, clentiazem, diltiazem, fendiline, gallopamil, mibefradil, prenylamine, semotiadil, terodiline, verapamil, amlodipine, aranidipine, barnidipine, benidipine, cilnidipine, efonidipine, elgodipine, felodipine, isradipine, lacidipine, lercanidipine, manidipine, nicardipine, nifedipine, nilvadipine, nimodipine, nisoldipine, nitrendipine, cinnarizine, flunarizine, lidoflazine, lomerizine, bencyclane, etafenone, fantofarone, and perhexiline.

Useful anticancer agents include, but are not limited to, acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alfa-2a; interferon alfa-2b; interferon alfa-n1 ; interferon alfa-n3; interferon beta-Ia; interferon gamma-Ib; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride. Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

Therapeutic agents useful for treating or preventing an addictive disorder include, but are not limited to, methadone, desipramine, amantadine; fluoxetine, buprenorphine, an opiate agonist, 3-phenoxypyridine, or a serotonin antagonist.

Useful anti-anxiety agents include, but are not limited to, benzodiazepines, such as alprazolam, chlordiazepoxide, clonazepam, clorazepate, diazepam, halazepam, lorazepam, oxazepam, and prazepam; non-benzodiazepine agents, such as buspirone; and tranquilizers, such as barbiturates.

Therapeutic agents useful for treating or preventing Parkinson's disease include, but are not limited to, carbidopa/levodopa, pergolide, bromocriptine, selegiline, amantadine, or trihexyphenidyl hydrochloride.

A compound of the present invention and another therapeutic agent can act additively or, more preferably, synergistically. In a preferred embodiment, a compound of the present invention is administered concurrently with another therapeutic agent. In one embodiment, a composition comprising an effective amount of a compound of the present invention and an effective amount of another therapeutic agent can be administered. Alternatively, a composition comprising an effective amount of a compound of the present invention and a different composition comprising an effective amount of another therapeutic agent can be concurrently administered. In another embodiment, an effective amount of a compound of the present invention is administered prior or subsequent to administration of an effective amount of another therapeutic agent. The two agents of the combination can be administered by the same or by different routes, depending on the condition being treated.

Generally, topical preparations contain from about 0.01% to about 100% by weight of the compound of the present invention, based upon 100% total weight of the topical preparation. In a preferred embodiment, topical preparations contain from about 0.1% to about 50 % by weight of the peptides, and more preferably from about 1.0 % to about 25% by weight of the peptides.

Generally, the pharmaceutical composition for parenteral administration contains from about 0.01 % to about 90 % by weight of the peptidomimetics of the present invention, based upon 100 % weight of total pharmaceutical composition. In a preferred embodiment, preparations for parenteral administration contain from about 0.1% to about 50% by weight of the peptidomimetics, and more preferably from about 1.0% to about 25% by weight of the peptidomimetics.

Generally, transdermal dosage forms contain from about 0.01% to about 100% by weight of the compound of the present invention, based upon 100% total weight of the dosage. In a preferred embodiment, transdermal preparations contain from about 0.1% to about 50% by weight of the compound, and more preferably from about 1.0% to about 25% by weight of the compound.

Finally, the preparations or compositions of the present invention may contain a compound of the present invention as about 50 % or more by weight of the active ingredient, preferably, about 75 % or more, more preferably, 90 % or more, 95 % or more, 99% or more, 99.5% or more, or most preferably 99.9% or more by weight of active ingredient.

The present invention further provides a kit, comprising at least one sterile container comprising an ORL-1 receptor ligand, or pharmaceutical composition, of the present invention, tailored according to its intended use. The kit optionally further comprises a second container comprising a sterile, pharmaceutically acceptable diluent useful to dilute or dissolve the active ingredient in the first container. Where the ORL-1 receptor ligand is an ORL-1 receptor agonist, the kit optionally further comprises a printed label and/or a set of printed instructions directing the use of the agonist or pharmaceutical composition to treat pain, anxiety, drug addiction, drug withdrawal and/or drug tolerance, or to enhance cognitive function, in a patient in need of such treatment. Where the ORL-1 receptor ligand is an ORL-1 receptor antagonist, the kit optionally further comprises a printed label and/or a set of printed instructions directing the use of the agonist or pharmaceutical composition to treat pain, drug addiction, drug withdrawal and/or drug tolerance in a patient in need of such treatment.

### EXAMPLES

The following Example illustrates the invention without limiting it.

### EXAMPLE 1

### Preparation and Analysis of Conformationally Constrained ORL-1 Receptor Agonists

**Abbreviations:** DIEA, N, N-diisopropylethylamine; DMF, N, N-dimethylformamide; Fmoc, 9-fluorenylmethyoxycarbonyl; HATU, N-((dimethylamino)-1H-1,2,3-triazolo(4,5-β)pyridin-1-ylmethylene)-N-methylmethan-aminium hexafluorophosphate N-oxide; HPLC, high performance liquid chromatography; Trt, triphenylmethyl(trityl); TFA, trifluoroacetic acid. Amino acids are in the L-configuration unless otherwise noted.

All chemicals and reagents were commercially available from different sources (Advanced ChemTech., Chem-Impex; AnaSpec; SynPep; Bachem). All peptides were prepared by manual solid-phase peptide synthesis using Fmoc chemistry. Standard protocol: 20% piperidine/DMF 7 minutes for Fmoc deblocking; 3-5 minutes for preactivation; 4 equivalents of Fmoc protected-amino acid, 4 equivalents of HATU and 8 equivalents of DIEA in DMF used for each coupling cycle; 30 minutes for each coupling unless otherwise noted. For coupling to α-methylalanine residue arbitrary double couplings were used (2 x 1h). For coupling to N-methylalanine residue arbitrary double couplings were used (2 x 3h). Tentagel Rink amide resin was used to make C-terminus amide peptide; Tentagel HMP loaded resin was used to make C-terminus acid peptide. All synthesized peptides and peptidomimetics were deprotected and cleaved from the resin using reagent K solution (TFA:phenol:H20:thioanisole:1,2-ethanedithio1;33:2:2:2:1) for 2 h at room temperature. The crude peptides or peptidomimetics were purified by Waters system Prep LC 4000. Peptide purity was determined by HPLC to be >95% purity, and peptide or peptidomimetic composition was determined by mass spectral analysis (MS, electrospray).

### Human ORL-1 Receptor Binding

All reagents were from Sigma (St. Louis, MO), unless otherwise noted. Membranes from recombinant HEK-293 cells expressing the human opioid receptor-like (ORL-1) receptor (Perkin Elmer, Boston, MA) were prepared by lysing cells in ice-cold hypotonic buffer (2.5 mM MgCl₂, 50 mM HEPES, pH 7.4) (10 ml, 10 cm dish) followed by homogenization with a tissue grinder/Teflon pestle. Membranes were collected by centrifugation at 30,000 x g for 15 minutes at 4°C and pellets resuspended in hypotonic buffer to a final concentration of 1-3 mg/ml. Protein concentrations were determined using the BioRad (Hercules, CA) protein assay reagent with bovine serum albumen as standard. Aliquots of the ORL-1 receptor membranes were stored at -80°C.

Radioligand dose-dependent binding assays used 0.1 nM [³H]-NC (NEN, Boston, MA) (87.7 Ci/mmole) with 5-10 µg membrane protein/well in a final volume of 500 µl binding buffer (10 mM MgCl₂, 1 mM EDTA, 5% DMSO, 50 mM HEPES, pH 7.4). Reactions were carried out in the absence and presence of increasing concentrations of unlabeled NC (American Peptide Co., Sunnyvale, CA) or its modified analogs. All reactions were conducted in 96-deep well polypropylene plates for 2 h at room temperature. Binding reactions were terminated by rapid filtration onto 96-well Unifilter GF/C filter plates (Packard, Meriden, CT) presoaked in 0.5% polyethyleneimine using a 96-well tissue harvester (Brandel, Gaithersburg, MD) followed by three filtration washes with 500 µl ice-cold binding buffer. Filter plates were subsequently dried at 50°C for 2-3 hours. BetaScint scintillation cocktail (Wallac, Turku, Finland) was added (50 µl/well). The data were analyzed using the one-site competition curve fitting functions in GraphPad PRISM, v. 3.0 (San Diego, CA).

### [³⁵S]GTPγS Functional Assay

[³⁵S]GTPγS functional assays were conducted using freshly thawed ORL-1 receptor membranes (see above). Assay reactions were prepared by sequentially adding the following reagents (final concentrations indicated): ORL-1 membrane protein (0.066 µg/µl), saponin (10 µg/ml), GDP (3 µM) and [³⁵S]GTPγS (0.20 nM; NEN) to binding buffer (100 mM NaCl, 10 mM MgCl₂, 20 mM HEPES, pH 7.4) on ice. The prepared membrane solution (190 µl/well) was transferred to 96-shallow well polypropylene plates containing 10 ml of 20x concentrated stock solutions of agonist (NC or NC analogs) prepared in DMSO. Plates were incubated for 30 minutes at room temperature with shaking. Reactions were terminated by rapid filtration onto 96-well Unifilter GF/B filter plates (Packard, Meriden, CT) using a 96-well tissue harvester (Brandel, Gaithersburg, MD) followed by three fitration washes with 200 µl ice-cold binding buffer (10 mM NaH₂PO₄, 10 mM Na₂HPO₄, pH 7.4). Filter plates were subsequently dried at 50°C for 2-3 hours. BetaScint scintillation cocktail (Wallac, Turku, Finland) was added (50 µl/well) and plates counted in a Packard Top-Count for 1 min/well. Data were analyzed using the sigmoidal dose-response curve fitting functions in GraphPad PRISM, v. 3.0.

### Results

Individual peptidomimetics, prepared as part of an initial systematic series, differed from NC only in that they contained N-or C^{α}-methyl alanine (NMA or AIB, respectively) as a replacement for the native Ala, either at position 7, 11, or 15. Like native NC, each of these peptidomimetics also contained free C-terminal carboxyl and N-terminal amino functionality. The human ORL-1 receptor affinities, EC₅₀ and Eₘₐₓ (as measured in the [³⁵S]GTPγS functional assay) for these peptides are presented in Table 1, below:

**TABLE 1**

| Peptide | Sequence | Kᵢ | EC₅₀ | Eₘₐₓ |
|---|---|---|---|---|
| NC | FGGFTGARKSARKLANQ (SEQ ID NO:1) | 0.3 ± 0.02 | 5.0 | 100 |
| I | FGGFTG(AIB)RKSARICLANQ (SEQ ID NO:32) | 0,1 ± .02 | 0.27 ± 0.08 | 97 |
| II | FGGFTGARKS(AIB)RKLANQ (SEQ ID NO: 33) | 0.48 ±.18 | 1.0 ± .1 | 96 |
| III | FGGFTGARKSARKL(AIB)NQ(SEQIDNO:34) | 0.15±.02 | 0.47 ±.14 | 91 |
| IV* | FGGFTG(NMA)RKSARKLANQ (SEQ ID NO:35) | 15.0 ± 4 | 96 ± 2 | 95 |
| V* | FGGFTGARKS(NMA)RKLANQ (SEQ ID NO:36) | 20.0 ± 8 | 407 ± 97 | 97 |
| VI* | FGGFTGARKSARKL(NMA)NQ (SEQ ID NO:37) | 1.1 ± .4 | 5.3 ± 1.1 | 93 |
| IA | FGGFTG(AIB)RKSARKLANQ-NH₂ (SEQ ID NO:38) | 0.05 ± 0.01 | 0.06 ± 0.03 | 82 |
| IIA | FGGFTGARKS(AIB)RKLANQ-NH₂ (SEQ ID NO:39) | 0.08 ± 0.01 | 0.14 ± 0.08 | 82 |
| IIIA | FGGFTGARKSARKL(AIB)NQ-NH₂ (SBQ ID NO:40) | 0.02 ± 0.01 | 0.2 ± 0.01 | 97 |
| IVA* | FGGFTG(NMA)RKSARKLANQ-NH₂ (SEQ ID NO:41) | 2.20 ± 0.7 | 10.5 ± 1.5 | 94 |
| VA* | FGGFTGARKS(NMA)RKLANQ-NH₂ (SEQ ID NO:42) | 6.70 ± 2.0 | 52 ± 8.0 | 91 |
| VIA* | FGGFTGARKSARKL(NMA)NQ-NH₂ (SEQ ID NO:43) | .06 ± 0.02 | .73 ± 0.11 | 87 |
| VIIA | FGGFTG(AIB)RKS(AIB)RKLANQ-NH₂ (SEQ ID NO:44) | .05 ± 0.01 | .08 ± 0.03 | 90 |

| | | | | |
|---|---|---|---|---|
| * comparative examples | | | | |

On the basis of the assay results summarized in Table 1, it is apparent that the AIB substitution, and therefore the imposed local helical conformation, is readily tolerated by the receptor regardless of the substitution at positions 7, 11, or 15. The measured binding affinities for these three peptidomimetics (I-III) range from 0.1 nM to 0.48 nM. These values are similar to the Kᵢ measured for NC (0.3 nM). Similarly, these three peptidomimetics (I-III) are full ORL-1 agonists having potencies and efficacies comparable to NC.

The NMA containing peptidomimetics from this series (IV-VI), although agonists, bind with significantly less affinity and are less potent than NC or the corresponding AIB-containing peptidomimetics. In particular, NMA-containing peptidomimetic (IV) binds to the ORL-1 receptor with 150-fold less affinity than the corresponding AIB-containing peptidomimetic (I), and is approximately 400-fold less potent in the functionality assay. A similar difference in potency is observed when comparing the NMA-containing peptidomimetic (V) to the AIB-containing peptidomimetic (II). Only a slight difference in binding and functionality activity (approximately 10-fold) was noted between the NMA- and AIB-containing peptidomimetics (III and VI, respectively). These data support the conclusion that local helical conformation, particularly about Ala⁷ and Ala¹¹, is preferred over extended conformation in the receptor-bound state. Conformational preference may be less critical about Ala¹⁵, although on the basis of these results, helix formation is still favored over extended ϕ,Ψ.

An analogous series of 6 peptidomimetics were prepared (Table 1, IA-VIA), which contained a C-terminal amide in place of the C-terminal carboxylate. The rationale behind this second series was based on the observation that at least a portion of the NC/ORL-1 binding energy results from electrostatic interactions between acidic residues (particularly in EL2) of the ORL-1 receptor and basic residues in the address segment of NC. Masking the acidic character of the C-terminal carboxylate as an amide might enhance the receptor interaction by presenting a more complementary electrostatic environment.

The results of ORL-1 receptor binding and functional assays are also presented in Table 1 for the C-terminal amide-containing peptides. Generally, the structure-activity relationship observed within this series parallels what was observed in the initial C-terminal carboxylate-containing series of peptidomimetics. One notable difference is that, while the trends are the same, the addition of the C-terminal amide enhances the affinity (3-10 fold improvement) and potency (5-10 fold improvement) with respect to the C-terminal carboxylate-containing peptides. Each of the AIB-containing, C-terminal amide peptides are more potent and bind the ORL-1 receptor with higher affinity than the native NC ligand.

AIB substitutions in this second series are conformationally preferable replacements for Ala⁷, Ala¹¹, or Ala¹⁵. In contrast, significant loss in relative affinity and potency was observed for the corresponding NMA substituted peptidomimetics, with the one exception of peptide VIA, which contains NMA at position 15. As was the case for the first series, these data also support the hypothesis that local helical conformation, particularly about Ala⁷ and Ala¹¹ is preferred over extended conformation in the receptor-bound form. Moreover, masking the acidic character of the native C-terminus as an amide, appears to enhance binding affinity and potency.

Based on the results described above and shown in Table 1, peptidomimetic VIIA was prepared and assayed. Peptidomimetic VIIA contains two AIB replacements in a single peptidomimetic, one at Ala⁷ and the other at Ala¹¹. In addition, this peptidomimetic contains a C-terminal amide. This double backbone constraint is likely to induce significant helical character into the secondary structure of the address segment of this peptidomimetic. Assay results on peptidomimetic VIIA show that it is a highly potent (EC₅₀=0.08 nM) and efficacious agonist with remarkable affinity (Kᵢ = 0.05 nM) for the human ORL-1 receptor. It has significantly higher affinity and is more potent than NC (Kᵢ = 0.3 nM; EC₅₀= 0.08 nM).

Without wishing to be bound by any particular theory, being a linear, highly polar heptadecapeptide, one would anticipate that in an aqueous environment, NC would be highly solvated by water and would rapidly inter-convert between many conformational states. At least one publication has demonstrated this by describing attempts to solve the solution conformation of NC using NMR techniques (Facchiano et al., Protein Eng. 1999, 10:893-99). The authors reported that NC adopts no preferential secondary structure in water because, on the NMR time scale, the rapid conformational inter-conversion appears random. However, it is likely that upon interaction with the ORL-1 receptor during, or just prior to the binding event, the NC peptide adopts a discreet, ordered structure that is highly complementary to the receptor binding site such that high affinity binding, and ultimately signaling, can readily occur.

Inspection of the primary sequence of NC, together with results obtained from two different secondary structure prediction algorithms led to the hypothesis that the native address segment of the NC peptide may adopt an alpha-helical conformation in the receptor-bound state. Results taken from secondary structure prediction algorithms, PHD and GOR IV, further support the concept (Figure 1).

This was experimentally tested by incorporating backbone constraints known to impose, either extended (ϕ, Ψ approximately 180°, 180°) or helical (ϕ, Ψ approximately -60°, -60°), structure into the sequence. Specifically, either Ala⁷, Ala¹¹, or Ala¹⁵ was replaced with NMA, to impose a local extended β structure, or with AIB to impose a local α helical structure.

All of the AIB-containing peptides have higher ORL-1 receptor affinity than the native NC ligand. Thus, one may conclude that in the receptor-bound state, NC adopts a helix (or helix-like structure) in the address segment. Due to the distribution of adjacent basic residues in this part of the sequence, the helix has significant amphipathic character. For illustrative purposes, Figure 2 shows a model of the ARKSARKLA (SEQ ID NO:45) segment of the NC message created by imposing standard ϕ, Ψ angles corresponding to alpha-helix into the backbone. No attempts were made to accurately model the side chain dihedral angles in this model so they appear artificially extended in Figure 2. It is clear, however, that Arg^{8,12} and Lys^{9,13} are situated on the same face of the proposed helix, and represent a highly complimentary electrostatic binding partner for the acidic residues in extra-cellular loop domain(s) of the receptor.

The introduction of these conformational constraints reduces the overall flexibility of the peptide, favoring a conformation that resembles the bio-active form. This entropic advantage manifests as a higher affinity, as measured by the thermodynamic equilibrium constant, Kᵢ. In contrast, NMA substitutions at either Ala⁷ or Ala¹¹ results in peptides that are less potent and bind with significantly lower affinity to the ORL-1 receptor. The imposed "extended" local conformation of these peptides must be less similar to the bio-active form of NC.

Another interesting observation from the series of peptidomimetics described herein is that affinities and potencies were always improved when the C-terminus of the peptide contained an amide, rather than a free carboxylate group. If one considers the proposal that has been made regarding the receptor-bound orientation of NC, then some possible electrostatic-based explanations emerge. In particular, it is presumed that the N-terminal FGGF, or message segment of NC binds to a site of the receptor that is primarily comprised of residues from the transmembrane (TM) domains, the hallmark of which is an electrostatic interaction between the N-terminal amino group of NC and the highly conserved aspartic acid residue (Asp¹³⁰) in TM3 of the receptor. All computer modeling studies reported thus far on the NC/ORL-1 complex incorporate this interaction as a key anchor point. If true, it would mean that the C-terminal receptor segment of NC is likely to be oriented toward, and therefore interacting with, one or more of the extra-cellular loop (EL) domains of the receptor. If the receptor-binding site that accommodates the message segment of NC is primarily an acidic environment, then the C-terminal carboxylate of NC maybe somewhat repulsive, since it is of the same charge. Transforming the C-terminal aid of NC to an amide, as described herein, may mask the charge such that it is more complementary to the electrostatic environment posed by the corresponding domain of the binding site.

In both series of peptidomimetics, the most modest contrast appears in results between the incorporation of NMA versus AIB at position 15, which is toward the C-terminal end. Specifically, in series one (Table 1) the difference in binding and functional potency was 10-fold, favoring the AIB-containing peptidomimetic (III) over the NMA-containing peptidomimetic (VI). The difference was even less in the corresponding series two peptidomimetics (IIIA and VIA). In that case, there was no apparent loss in binding affinity and the potency loss was only 3-fold. Thus, the C-terminal part of NC does not appear to make a substantial energetic contribution to binding, and conformational preference is minimal. Indeed, the results provide evidence that, in the vicinity of Ala¹⁵, there is considerable tolerance for different ligand conformation since both the Ala¹⁵, and NMA¹⁵ peptides bind similarly.

One final point of interest is the recent report that NC (1-13)-CONH₂ is also a full agonist with affinity similar to NC (Guerrini et al. J. Med. Chem. 1997, 40:1789-1793). This peptidomimetic is effectively a C-terminal truncation peptide, missing four residues from the end of the native sequence (LANQ, corresponding to residues 14-17 of SEQ ID NO:1). This peptidomimetic further exemplifies the lack of conformational stringency, or required binding energy contribution, associated with the C-terminal portion of NC.

## Claims

1. An ORL-1 receptor ligand comprising
a C-terminal address segment from the native nociceptin (NC) ligand, having the amino acid sequence of SEQ ID NO: 3, which address segment has heen modified by the substitution of one or more amino acid residues with one or more α-helix promoting amino acids (x_{α}) or with one or more α-helix promoting amino acid analogs (x_{α}), wherein the at least one amino acid analog is AIB, wherein the address segment comprises a sequence selected from the group consisting of:
(X_{α})-RKS-A-RKL-A-NQ(SEQ ID NO:4);
A-RKS-(X_{α})-RKL-A-NQ (SEQ ID NO:5);
A-RKS-A-RKL-(X_{α})-NQ (SEQ ID NO:6);
(X_{α})-RKS-(X_{α})-RKL-A-NQ (SEQ ID NO:7);
(X_{α})-RKS-A-RKL-(X_{α})-NQ (SEQ ID NO:8);
A-RKS-(X_{α})-RKL-(X_{α})-NQ(SEQ ID NO:9); and
(X_{α})-RKS-(X_{α})-RKL-(X_{α})-NQ (SEQ ID NO:10);
wherein the ligand further comprises an N-terminal message segment from a native NC ligand, and phamaceutically acceptable salts and solvates thereof.

2. An ORL-1 receptor ligand comprising
a C-terminal address segment from the native nociceptin (NC) ligand, having the amine acid sequence of SEQ ID NO: 3, which address segment has been modified by the substitution of one or more amino acid residues with one or more
α-helix promoting amino acids (X_{α}) or with one or more α-helix promoting amino acid analogs (x_{α}), wherein the at least one amino acid analog is AIB, wherein the address segment comprises a sequence selected from the group consisting of:
A-RKS-(X_{α})-RK (SEQ ID NO:48);
(X_{α})-RKS-A-RK (SEQ ID NO:49); and
(X_{α})-RKS-(X_{α})-RK (SEQ ID NO:50);
wherein the ligand further comprises an N-terminal message segment from a native NC ligand,
and pharmaceutically acceptable salts and solvates thereof.

3. An ORL-1 receptor ligand according to claim 1, which comprises a sequence selected from the group consisting of:
FGGF-TG-(X_{α})-RKS-A-RKL-A-NQ (SEQ ID NO:18);
FGGF-TG-A-RKS-(X_{α})-RKL-A-NQ (SEQ ID NO:19);
FGGF-TG-A-RKS-A-RKL-(X_{α})-NQ (SEQ ID NO:20);
FGGF-TG-(X_{α})-RKS-(X_{α})-RKL-A-NQ (SEQ ID NO:21);
FGGF-TG-(X_{α})-RKS-A-RKL-(X_{α})-NQ (SEQ ID NO:22;
FGGF-TG-A-RKS-(X_{α})-RKL-(X_{α})-NQ (SEQ ID NO:23); and
FGGF-TG-(X_{α})-RKS-(X_{α})-RKL-(X_{α})-NQ (SEQ ID NO:24);
wherein each X_{α} is an independently selected same or different alpha-helix inducing amino acid or alpha-helix inducing amino acid analog wherein the at least one amino acid analog is AIB; and pharmaceutically acceptable salts and solvates thereof.

4. An ORL-1 receptor ligand according to claim 2, which comprises a sequence selected from the group consisting of:
FGGF-TG-A-RKS-(X_{α})-RK-NH₂ (SEQ ID NO:51);
FGGF-TG-(X_{α})-RKS-A-RK-NH₂ (SEQ ID NO:52); and
FGGF-TG-( X_{α})-RKS-( X_{α})-RK-NH₂ (SEQ ID NO:53);
wherein each X_{α} is an independently selected same or different alpha-helix inducing amino acid or alpha-helix inducing amino acid analog wherein the at least one amino acid analog is AIB; and pharmaceutically acceptable salts and solvates thereof.

5. The ORL-1 receptor ligand of any of the preceding claims, wherein the carboxyl group on the C-terminal amino acid residue is substituted with a moiety selected from the group consisting of an ester, ketone, substituted amide, unsubstituted amide, ether, and amine.

6. Use of the ORL-ligand of any of the proceeding claims in the manufacture of a medicament for treating pain in a patient in need of such treatment, wherein the treatment comprises administering to the patient an analgesic effective amount of the ORL-ligand.

7. Use of the ORL-ligand of any of claims 1-5 in the manufacture of a medicament for treating anxiety, drug addiction, drug withdrawal or drug tolerance in a patient in need of such treatment, wherein the treatment comprises-administering to the patient a therapeutically effective amount of the ORL-ligand.

8. Use of the ORL-ligand of any of claims 1-5 in the manufacture of a medicament for enhancing cognitive function in a patient in need of such treatment, wherein the treatment comprises administering to the patient a therapeutically effective amount the ORL-ligand.

9. Use of the ORL-ligand of any of claims 1-5 in the manufacture of a medicament for modulating a pharmacological response from an ORL-1 receptor, wherein modulating the pharmacological response comprises contacting the ORL-1 receptor with the ORL-1 receptor ligand and wherein the medicament can be used to treat pain, anxiety, drug addiction, drug withdrawal or drug tolerance, or to enhance cognitive function.

10. A pharmaceutical composition comprising the ORL-1 receptor ligand of any of claims 1-5, which ORL-1 receptor ligand is an agonist, combined with a pharmaceutically acceptable carrier.

11. A kit, comprising a sterile container comprising the pharmaceutical composition of claim 10, and a printed label or a set of printed instructions directing the use of the agonist to treat pain, anxiety, drug addiction, drug withdrawal or drug tolerance, or to enhance cognitive function, in a patient in need of such treatment.

12. A method of screening for compounds capable of modulating ORL-1 receptor activity, comprising:
(a) obtaining an ORL-1 ligand according to claim 1;
(b) combining the ligand of step (a) with an ORL-1 receptor, or a ligand-binding portion thereof, under conditions that permit or induce formation of a ligand-receptor complex;
(c) exposing the ligand-receptor complex of step (b) to a test compound; and
(d) detecting: (i) displacement of the ligand from the ligand-receptor complex, or (ii) a change in ORL-1 receptor activity;
wherein a test compound that is determined to displace the ORL-1 ligand from the ligand-receptor complex or to cause a change in ORL-1 receptor activity is identified as a compound capable of modulating ORL-1 receptor activity.

13. The ligand of claim 1, wherein the ligand binds to the receptor with a binding Kᵢ of at least approximately the binding Kᵢ of a native NC ligand.

14. The ligand of claim 1, wherein the ligand is a full agonist, partial agonist, full antagonist, partial antagonist, or inverse agonist.

15. A method of screening for compounds capable of interacting with an ORL-1 receptor, comprising:
(a) obtaining a compound according to claim 1.
(b) testing the compound for its ability to bind to an ORL-1 receptor, or for its ability to agonize or antagonize the ORL-1 receptor;
wherein a compound determined to bind to, agonize, or antagonize, the ORL-1 receptor is identified as a compound capable of interacting with the ORL-1 receptor.

16. An ORL-1 receptor ligand of any of claims 1-5 for use in the treatment of pain, anxiety, drug addiction, drug withdrawal or drug tolerance, or for enhancing cognitive function in a patient in need of such treatment, wherein the treatment comprises administering to the patient an effective amount of the ORL-ligand.

## Patentansprüche

1. ORL-1-Rezeptor-Ligand, enthaltend ein C-terminales Adressen-Segment des nativen Nociceptin (NC)-Liganden mit der Aminosäuresequenz der SEQ ID NO. 3, welches modifiziert wurde durch die Substitution eines oder mehrerer Aminosäurereste mit einer oder mehreren α-Helix-induzierenden Aminosäuren (X_{α}) oder mit einem oder mehreren α-Helix-induzierenden Aminosäureanaloga (X_{α}), wobei das mindestens eine Aminosäureanalogon AIB ist, wobei das Adressen-Segment eine Sequenz enthält, die ausgewählt ist aus der Gruppe bestehend aus:
(X_{α})-RKS-A-RKL-A-NQ (SEQ ID NO:4);
A-RKS-(X_{α})-RKL-A-NQ (SEQ ID NO:5);
A-RKS-A-RKL-(X_{α})-NQ (SEQ ID NO:6);
(X_{α})-RKS-(X_{α})-RKL-A-NQ (SEQ ID NO:7);
(X_{α})-RKS-A-RKL-(X_{α})-NQ (SEQ ID NO:8);
A-RKS-(X_{α})-RKL-(X_{α})-NQ (SEQ ID NO:9); und
(X_{α})-RKS-(X_{α})-RKL-(X_{α})-NQ (SEQ ID NO:10);
wobei der Ligand ferner ein N-terminales Nachrichten-Segment eines nativen NC-Liganden enthält, sowie pharmazeutisch akzeptable Salze und Solvate davon.

2. ORL-1-Rezeptor-Ligand, enthaltend ein C-terminales Adressen-Segment des nativen Nociceptin (NC)-Liganden mit der Aminosäuresequenz der SEQ ID NO. 3, welches modifiziert wurde durch die Substitution eines oder mehrerer Aminosäurereste mit einer oder mehreren α-Helix-induzierenden Aminosäuren (X_{α}) oder mit einem oder mehreren α-Helix-induzierenden Aminosäureanaloga (X_{α}), wobei das mindestens eine Aminosäureanalogon AIB ist, wobei das Adressen-Segment eine Sequenz enthält, die ausgewählt ist aus der Gruppe bestehend aus:
A-RKS-(X_{α})-RK (SEQ ID NO:48);
(X_{α})-RKS-A-RK (SEQ ID NO:49); und
(X_{α})-RKS-(X_{α})-RK (SEQ ID NO:50);
wobei der Ligand ferner ein N-terminales Nachrichten-Segment eines nativen NC-Liganden enthält, sowie pharmazeutisch akzeptable Salze und Solvate davon.

3. ORL-1-Rezeptor-Ligand gemäß Anspruch 1, enthaltend eine Sequenz, die ausgewählt ist aus der Gruppe bestehend aus:
FGGF-TG-(X_{α})-RKS-A-RKL-A-NQ (SEQ ID NO:18);
FGGF-TG-A-RKS-(X_{α})-RKL-A-NQ (SEQ ID NO:19);
FGGF-TG-A-RKS-A-RKL-(X_{α})-NQ (SEQ ID NO:20);
FGGF-TG-(X_{α})-RKS-(X_{α})-RKL-A-NQ (SEQ ID NO:21);
FGGF-TG-(X_{α})-RKS-A-RKL-(X_{α})-NQ (SEQ ID NO:22;
FGGF-TG-A-RKS-(X_{α})-RKL-(X_{α})-NQ (SEQ ID NO:23); und
FGGF-TG-(X_{α})-RKS-(X_{α})-RKL-(X_{α})-NQ (SEQ ID NO:24);
wobei jedes X_{α} eine unabhängig voneinander ausgewählte gleiche oder verschiedene α-Helixinduzierende Aminosäure oder ein unabhängig voneinander ausgewähltes gleiches oder verschiedenes α-Helix-induzierendes Aminosäureanalogon ist, wobei das mindestens eine Aminosäureanalogon AIB ist; sowie pharmazeutisch akzeptable Salze und Solvate davon.

4. ORL-1-Rezeptor-Ligand gemäß Anspruch 2, enthaltend eine Sequenz, die ausgewählt ist aus der Gruppe bestehend aus:
FGGF-TG-A-RKS-(X_{α})-RK-NH₂ (SEQ ID NO:51);
FGGF-TG-(X_{α})-RKS-A-RK-NH₂ (SEQ ID NO:52); und
FGGF-TG-(X_{α})-RKS-(X_{α})-RK-NH₂ (SEQ ID NO:53);
wobei jedes X_{α} eine unabhängig voneinander ausgewählte gleiche oder verschiedene α-Helixinduzierende Aminosäure oder ein unabhängig voneinander ausgewähltes gleiches oder verschiedenes α-Helix-induzierendes Aminosäureanalogon ist, wobei das mindestens eine Aminosäureanalogon AIB ist; sowie pharmazeutisch akzeptable Salze und Solvate davon.

5. ORL-1-Rezeptor-Ligand gemäß einem der vorangehenden Ansprüche, wobei die Carboxylgruppe am C-terminalen Aminosäurerest mit einer Gruppe substituiert ist, die ausgewählt ist aus der Gruppe bestehend aus einem Ester, Keton, substituierten Amid, nicht substituierten Amid, Ether und Amin.

6. Verwendung des ORL-Liganden gemäß einem der vorangehenden Ansprüche zur Herstellung eines Medikaments für die Behandlung von Schmerzen eines behandlungsbedürftigen Patienten, wobei die Behandlung das Verabreichen einer analgetisch wirksamen Menge des ORL-Liganden an den Patienten umfasst.

7. Verwendung des ORL-Liganden gemäß einem der Ansprüche 1 - 5 zur Herstellung eines Medikaments für die Behandlung von Angstzuständen, von Abhängigkeitssyndromen, von Entzugssyndromen, oder von pharmakologischer Toleranz eines behandlungsbedürftigen Patienten, wobei die Behandlung das Verabreichen einer therapeutisch wirksamen Menge des ORL-Liganden an den Patienten umfasst.

8. Verwendung des ORL-Liganden gemäß einem der Ansprüche 1 - 5 zur Herstellung eines Medikaments für die Verbesserung der kognitiven Fähigkeiten eines behandlungsbedürftigen Patienten, wobei die Behandlung das Verabreichen einer therapeutisch wirksamen Menge des ORL-Liganden an den Patienten umfasst.

9. Verwendung des ORL-Liganden gemäß einem der Ansprüche 1 - 5 zur Herstellung eines Medikaments für die Modulation der pharmakologischen Wirkung eines ORL-1-Rezeptors, wobei die Modulation der pharmakologischen Wirkung das In-Kontakt-Bringen des ORL-1-Rezeptors mit dem ORL-1-Rezeptor-Liganden umfasst und wobei das Medikament zur Behandlung von Schmerzen, von Angstzuständen, von Abhängigkeitssyndromen, von Entzugssyndromen, oder von pharmakologischer Toleranz, oder für die Verbesserung der kognitiven Fähigkeiten verwendet werden kann.

10. Pharmakologische Zusammensetzung enthaltend den ORL-1-Rezeptor-Liganden gemäß einem der Ansprüche 1 - 5, wobei der ORL-1-Rezeptor-Ligand ein Agonist ist und mit einem pharmazeutisch akzeptablen Träger kombiniert ist.

11. Kit, umfassend einen sterilen Behälter, der die pharmazeutische Zusammensetzung gemäß Anspruch 10 umfasst, sowie ein bedrucktes Etikett oder ein Set von gedruckten Anweisungen mit einer Anleitung für die Verwendung des Agonisten zur Behandlung von Schmerzen, von Angstzuständen, von Abhängigkeitssyndromen, von Entzugssyndromen, oder von pharmakologischer Toleranz, oder für die Verbesserung der kognitiven Fähigkeiten eines behandlungsbedürftigen Patienten.

12. Verfahren zum Aussieben von Verbindungen, die die ORL-1-Rezeptor-Aktivität modulieren können, umfassend:
(a) das Bereitstellen eines ORL-1-Liganden gemäß Anspruch 1;
(b) das Kombinieren des Liganden aus Schritt (a) mit einem ORL-1-Rezeptor oder eines Liganden-bindenden Teils davon, unter Bedingungen, die die Bildung eines Liganden-Rezeptor-Komplexes erlauben oder induzieren;
(c) das In-Kontakt-Bringen des Liganden-Rezeptor-Komplexes aus Schritt (b) mit einer Testverbindung; und
(d) das Ermitteln: (i) Verdrängung des Liganden aus dem Liganden-Rezeptor-Komplex oder (ii) Veränderung der ORL-1-Rezeptor-Aktivität;
wobei eine Testverbindung, von der ermittelt wurde, dass sie den ORL-1-Liganden aus dem Liganden-Rezeptor-Komplex verdrängt oder eine Veränderung in der ORL-1-Rezeptor-Akivität bewirkt, als eine Verbindung identifiziert wird, die die ORL-1-Rezeptor-Aktivität modulieren kann.

13. Ligand gemäß Anspruch 1, wobei der Ligand an den Rezeptor mit einer Bindungskonstante Kᵢ bindet, welche mindestens ungefähr der Bindungskonstante Kᵢ eines nativen NC-Liganden entspricht.

14. Ligand gemäß Anspruch 1, wobei der Ligand ein vollständiger Agonist, partieller Agonist, vollständiger Antagonist, partieller Antagonist oder inverser Agonist ist.

15. Verfahren zum Aussieben von Verbindungen, die mit einem ORL-1-Rezeptor interagieren können, umfassend:
(a) das Bereitstellen einer Verbindung gemäß Anspruch 1;
(b) das Testen der Verbindung bezüglich ihrer Fähigkeit, an einen ORL-1-Rezeptor zu binden oder agonistisch oder antagonistisch auf den ORL-1-Rezeptor zu wirken;
wobei eine Verbindung, von der ermittelt wurde, dass sie am ORL-1-Rezeptor bindet, oder agonistisch oder antagonistisch auf den ORL-1-Rezeptor wirkt, als eine Verbindung identifiziert wird, die mit dem ORL-1-Rezeptor interagieren kann.

16. ORL-1-Rezeptor-Ligand gemäß einem der Ansprüche 1 - 5 zur Verwendung in der Behandlung von Schmerzen, von Angstzuständen, von Abhängigkeitssyndromen, von Entzugssyndromen, oder von pharmakologischer Toleranz, oder in der Verbesserung der kognitiven Fähigkeiten eines behandlungsbedürftigen Patienten, wobei die Behandlung das Verabreichen einer therapeutisch wirksamen Menge des ORL-Liganden an den Patienten umfasst.

## Revendications

1. Ligand du récepteur ORL-1 comprenant
un segment d'adresse C-terminal provenant du ligand natif de nociceptine (NC) ayant la séquence d'acide aminé de SEQ ID NO :3, lequel segment d'adresse a été modifié par la substitution d'un ou plusieurs résidus d'acide aminé avec un ou plusieurs acides aminés (Xα) favorisant l'hélice-α ou avec un ou plusieurs analogues d'acide aminé (Xα) favorisant l'hélice-α, dans lequel le au moins un analogue d'acide aminé est AIB, dans lequel le segment d'adresse comprend une séquence sélectionnée parmi le groupe consistant en :
(X_{α})-RKS-A-RKL-A-NQ (SEQ ID NO:4);
A-RKS-(X_{α})-RKL-A-NQ (SEQ ID NO:5);
A-RKS-A-RKL-(X_{α})-NQ (SEQ ID NO:6);
(X_{α})-RKS-(X_{α})-RKL-A-NQ (SEQ ID NO:7);
(X_{α})-RKS-A-RKL-(X_{α})-NQ (SEQ ID NO:8);
A-RKS-(X_{α})-RKL-(X_{α})-NQ (SEQ ID NO:9); et
(X_{α})-RKS-(X_{α})-RKL-(X_{α})-NQ (SEQ ID NO:10);
dans lequel le ligand comprend en outre un segment de message N-terminal provenant d'un ligand natif de NC,
et des sels pharmaceutiquement acceptables et des solvates de ceux-ci.

2. Ligand du récepteur ORL-1 comprenant
un segment d'adresse C-terminal provenant du ligand natif de nociceptine (NC) ayant la séquence d'acide aminé de SEQ ID NO :3, lequel segment d'adresse a été modifié par la substitution d'un ou plusieurs résidus d'acide aminé avec un ou plusieurs acides aminés (Xα) favorisant l'hélice-α ou avec un ou plusieurs analogues d'acide aminé (Xα) favorisant l'hélice-α, dans lequel le au moins un analogue d'acide aminé est AIB, dans lequel le segment d'adresse comprend une séquence sélectionnée parmi le groupe consistant en :
**A-RKS-(X**_{α}**)-RK (SEQ ID NO:48);**
**(X**_{α})-**RKS-A-RK (SEQ ID NO:49); et**
**(X**_{α}**)-RKS-(X**_{α}**)-RK (SEQ ID NO:50);**
dans lequel le ligand comprend en outre un segment de message N-terminal provenant d'un ligand natif de NC,
et des sels pharmaceutiquement acceptables et des solvates de ceux-ci.

3. Ligand du récepteur ORL-1 selon la revendication 1, qui comprend une séquence sélectionnée parmi le groupe consistant en :
FGGF-TG-(X_{α})-RKS-A-RKL-A-NQ (SEQ ID NO:18);
FGGF-TG-A-RKS-(X_{α})-RKL-A-NQ (SEQ ID NO:19);
FGGF-TG-A-RKS-A-RKL-(X_{α})-NQ (SEQ ID NO:20);
FGGF-TG-(X_{α})-RKS-(X_{α})-RKL-A-NQ (SEQ ID NO:21);
FGGF-TG-(X_{α})-RKS-A-RKL-(X_{α})-NQ (SEQ ID NO:22;
FGGF-TG-A-RKS-(X_{α})-RKL-(X_{α})-NQ (SEQ ID NO:23); et
FGGF-TG-(X_{α})-RKS-(X_{α})-RKL-(X_{α})-NQ (SEQ ID NO:24);
dans laquelle chaque Xα est un même ou un différent acide aminé induisant l'hélice-α ou un analogue d'acide aminé induisant l'hélice-α, sélectionné indépendamment, dans laquelle le au moins un analogue d'acide aminé est AIB ; et des sels pharmaceutiquement acceptables et des solvates de ceux-ci.

4. Ligand du récepteur ORL-1 selon la revendication 2, qui comprend une séquence sélectionnée parmi le groupe consistant en :
FGGF-TG-A-RKS-(X_{α})-RK-NH₂ (SEQ ID NO:51);
FGGF-TG-(X_{α})-RKS-A-RK-NH₂ (SEQ ID NO:52); et
FGGF-TG-( X_{α})-RKS-( X_{α})-RK-NH₂ (SEQ ID NO:53);
dans laquelle chaque Xα est un même ou un différent acide aminé induisant l'hélice-α ou un analogue d'acide aminé induisant l'hélice-α, sélectionné indépendamment, dans laquelle le au moins un analogue d'acide aminé est AIB ; et des sels pharmaceutiquement acceptables et des solvates de ceux-ci.

5. Ligand du récepteur ORL-1 selon l'une quelconque des revendications précédentes, dans lequel le groupe carboxyle sur le résidu d'acide aminé C-terminal est substitué avec un groupement sélectionné parmi le groupe consistant en un ester, une cétone, un amide substitué, un amide non substitué, un éther er un amine.

6. Utilisation du ligand ORL-1 selon l'une quelconque des revendications précédentes pour la fabrication d'un médicament pour le traitement de la douleur dans un patient ayant besoin d'un tel traitement, dans laquelle le traitement comprend l'administration d'une quantité analgésique effective du ligand ORL.

7. Utilisation du ligand ORL-1 selon l'une quelconque des revendications 1-5 pour la fabrication d'un médicament pour le traitement de l'anxiété, la toxicomanie, le manque de drogue ou la tolérance de drogue dans un patient ayant besoin d'un tel traitement, dans laquelle le traitement comprend l'administration d'une quantité therapeutiquement effective du ligand ORL.

8. Utilisation du ligand ORL-1 selon l'une quelconque des revendications 1-5 pour la fabrication d'un médicament pour améliorer la fonction cognitive dans un patient ayant besoin d'un tel traitement, dans laquelle le traitement comprend l'administration au patient une quantité therapeutiquement effective du ligand ORL.

9. Utilisation du ligand ORL-1 selon l'une quelconque des revendications 1-5 pour la fabrication d'un médicament pour la modulation d'une réaction pharmacologique provenant d'un récepteur ORL-1, dans laquelle la modulation de la réaction pharmaceutique comprend le contact du récepteur ORL-1 avec le ligand du récepteur OR-L-1 et dans laquelle le médicament peut être utilisé pour le traitement de l'anxiété, la toxicomanie, le manque de drogue ou la tolérance de drogue ou pour l'amélioration de la fonction cognitive.

10. Composition pharmaceutique comprenant le ligand du récepteur ORL-1 selon l'une quelconque des revendications 1-5, lequel ligand du récepteur ORL-1 est un agoniste, en association avec un véhicule pharmaceutiquement acceptable.

11. Kit comprenant un récipient stérile comprenant la composition pharmaceutique selon la revendication 10 et une étiquette imprimée ou une série d'instructions imprimées dirigeant l'utilisation de l'agoniste pour le traitement de la douleur, de l'anxiété, la toxicomanie, manque de drogue ou tolérance de drogue ou pour l'amélioration de la fonction cognitive dans un patient ayant besoin d'un tel traitement.

12. Procédé de criblage pour des composés capables de moduler l'activité du récepteur ORL-1, comprenant :
(a) l'obtention d'un ligand ORL-1 selon la revendication 1 ;
(b) l'association du ligand de l'étape (a) avec un récepteur ORL-1 ou une partie de celui-ci reliant le ligand, sous des conditions permettant ou induisant la formation d'une complexe de ligand-récepteur ;
(c) l'exposition du complexe ligand-récepteur de l'étape (b) à un composé d'essai ; et
(d) la détection : (i) du déplacement du ligand du complexe ligand-récepteur, ou (ii) d'une modification de l'activité du récepteur ORL ;
dans lequel un composé d'essai qui est prévu pour le déplacement du ligand ORL-1 du complexe ligand-récepteur ou pour la provocation d'une modification de l'activité du récepteur ORL-1 est identifié comme un composé capable de moduler l'activité du récepteur ORL-1.

13. Ligand selon la revendication 1, dans lequel le ligand s'attache au récepteur avec une constante de liaison Kᵢ d' au moins environs celle d'un ligand natif de NC.

14. Ligand selon la revendication 1, dans lequel le ligand est un agoniste complet, agoniste partiel, antagoniste complet ou un agoniste inverse.

15. Procédé de criblage pour des composés capables d'interagir avec un récepteur ORL-1, comprenant :
(a) l'obtention d'un compose selon la revendication 1 ;
(b) l'essai du composé pour sa capacité de lier à un récepteur ORL-1 ;
dans lequel un composé qui est prévu pour la liaison, l'agonisation ou l'antagonisation du récepteur ORL-1 est identifié comme un composé capable d'interagir avec le récepteur ORL-1.

16. Ligand du récepteur ORL-1 selon l'une des revendications 1-5 pour l'utilisation chez le traitement de la douleur, l'anxiété, la toxicomanie, le manque de drogue ou la tolérance de drogue ou pour l'amélioration de la fonction cognitive dans un patient ayant besoin d'un tel traitement, dans lequel le traitement comprend l'administration d'une quantité effective du ligand ORL-1 au patient.


### SEQUENCE LISTING

<110> Euro-Celtique S.A.
   Kyle, Donald
<120> CONFORMATIONALLY CONSTRAINED PEPTIDES THAT BIND THE ORL-1 RECEPTOR
<130> 2755/2K483-WOO
<150> US 60/376,745
   <151> 2002-04-29
<160> 56
<170> PatentIn version 3.1
<210> 1
   <211> 17
   <212> PRT
   <213> Homo sapiens
<400> 1
   Phe Gly Gly Phe Thr Gly Ala Arg Lys Ser Ala Arg Lys Leu Ala Asn
   1               5                   10                  15
   Gln
<210> 2
   <211> 4
   <212> PRT
   <213> Homo sapiens
<400> 2
   Phe Gly Gly Phe
   1
<210> 3
   <211> 13
   <212> PRT
   <213> Homo sapiens
<400> 3
   Thr Gly Ala Arg Lys Ser Ala Arg Lys Leu Ala Asn Gln
   1               5                   10
<210> 4
   <211> 11
   <212> PRT
   <213> Artificial Sequence .
<220>
   <223> ORL-1 receptor ligand
<220>
   <221> MISC_FEATURE
   <222> (1)..(11)
   <223> where X is an alpha helix promoter
<400> 4
   Xaa Arg Lys Ser Ala Arg Lys Leu Ala Asn Gln
   1               5                   10
<210> 5
   <211> 11
   <212> PRT
   <213> Artificial Sequence
<220>
   <223> ORL-1 receptor ligand
<220>
   <221> MISC_FEATURE
   <222> (1)..(11)
   <223> where X is an alpha helix promoter
<400> 5
   Ala Arg Lys Ser Xaa Arg Lys Leu Ala Asn Gln
   1 5 10
<210> 6
   <211> 11
   <212> PRT
   <213> Artificial Sequence
<220>
   <223> ORL-1 receptor ligand
<220>
   <221> MISC_FEATURE
   <222> (1).. (11)
   <223> where X is an alpha helix promoter
<400> 6
   Ala Arg Lys Ser Ala Arg Lys Leu Xaa Asn Gln
   1 5 10
<210> 7
   <211> 11
   <212> PRT
   <213> Artificial Sequence
<220>
   <223> ORL-1 receptor ligand
<220>
   <221> MISC_FEATURE
   <222> (1)..(11)
   <223> where X is an alpha helix promoter
<400> 7
   Xaa Arg Lys Ser Xaa Arg Lys Leu Ala Asn Gln
   1 5 10
<210> 8
   <211> 11
   <212> PRT
   <213> Artificial Sequence
<220>
   <223> ORL-1 receptor ligand
<220>
   <221> MISC_FEATURE
   <222> (1)..(11)
   <223> where X is an alpha helix promoter
<400> 8
   Xaa Arg Lys Ser Ala Arg Lys Leu Xaa Asn Gln
   1               5                   10
<210> 9
   <211> 11
   <212> PRT
   <213> Artificial Sequence
<220>
   <223> ORL-1 receptor ligand
<220>
   <221> MTSC_FEATURE
   <222> (1)..(11)
   <223> where X is an alpha helix promoter
<400> 9
   Ala Arg Lys Ser Xaa Arg Lys Leu Xaa Asn Gln
   1               5                   10
<210> 10
   <211> 11
   <212> PRT
   <213> Artificial Sequence
<220>
   <223> ORL-1 receptor ligand
<220>
   <221> MISC_FEATURE
   <222> (1)..(11)
   <223> where X is an alpha helix promoter
<400> 10
   Xaa Arg Lys Ser Xaa Arg Lys Leu Xaa Asn Gln
   1               5                   10
<210> 11
   <211> 11
   <212> PRT
   <213> Artificial Sequence
<220>
   <223> ORL-1 receptor ligand
<220>
   <221> MISC_FEATURE
   <222> (1)..(11)
   <223> where X is an extended beta-strand promoter (comparative)
<400> 11
   Xaa Arg Lys Ser Ala Arg Lys Leu Ala Asn Gln
   1               5                   10
<210> 12
   <211> 11
   <212> PRT
   <213> Artificial Sequence
<220>
   <223> ORL-1 receptor ligand
<220>
   <221> MTSC_FEATURE
   <222> (1)..(11)
   <223> where X is an extended beta-strand promoter (comparative)
<400> 12
   Ala Arg Lys Ser Xaa Arg Lys Leu Ala Asn Gln
   1               5                   10
<210> 13
   <211> 11
   <212> PRT
   <213> Artificial Sequence
<220>
   <223> ORL-1 receptor ligand
<220>
   <221> MISC_FEATURE
   <222> (1)..(11) an extended beta-strand promoter (comparative)
   <223> where X is an extended beta-strand promoter
<400> 13
   Ala Arg Lys Ser Ala Arg Lys Leu Xaa Asn Gln
   1               5                   10
<210> 14
   <211> 11
   <212> PRT
   <213> Artificial Sequence
<220>
   <223> ORL-1 receptor ligand
<220>
   <221> MISC_FEATURE
   <222> (1)..(11)
   <223> where X is an extended beta-strand promoter (comparative)
<400> 14
   Xaa Arg Lys Ser Xaa Arg Lys Leu Ala Asn Gln
   1 5 10
<210> 15
   <211> 11
   <212> PRT
   <213> Artificial Sequence
<220>
   <223> ORL-1 receptor ligand
<220>
   <221> MISC_FEATURE
   <222> (1)..(11)
   <223> where X is an extended beta-strand promoter (comparative)
<400> 15
   Xaa Arg Lys Ser Ala Arg Lys Leu Xaa Asn Gln
   1 5 10
<210> 16
   <211> 11
   <212> PRT
   <213> Artificial Sequence
<220>
   <223> ORL-1 receptor ligand
<220>
   <221> MISC_FEATURE
   <222> (1)..(11)
   <223> where X is an extended beta-strand promoter (comparative)
<400> 16
   Ala Arg Lys Ser Xaa Arg Lys Leu Xaa Asn Gln
   1 5 10
<210> 17
   <211> 11
   <212> PRT
   <213> Artificial Sequence
<220>
   <223> ORL-1 receptor ligand
<220>
   <221> MISC_FEATURE
   <222> (1)..(11)
   <223> where X is an extended beta-strand promoter (comparative)
<400> 17
   Xaa Arg Lys Ser Xaa Arg Lys Leu Xaa Asn Gln
   1 5 10
<210> 18
   <211> 17
   <212> PRT
   <213> Artificial Sequence
<220>
   <223> ORL-1 receptor ligand
<220>
   <221> MISC_FEATURE
   <222> (1)..(17)
   <223> where X is an alpha helix promoter
<400> 18
   Phe Gly Gly Phe Thr Gly Xaa Arg Lys Ser Ala Arg Lys Leu Ala Asn
   1 5 10 15
   Gln
<210> 19
   <211> 17
   <212> PRT
   <213> Artificial Sequence
<220>
   <223> ORL-1 receptor ligand
<220>
   <221> MISC_FEATURE
   <222> (1)..(17)
   <223> where X is an alpha helix promoter
<400> 19
   Phe Gly Gly Phe Thr Gly Ala Arg Lys Ser Xaa Arg Lys Leu Ala Asn
   1 5 10 15
   Gln
<210> 20
   <211> 17
   <212> PRT
   <213> Artificial Sequence
<220>
   <223> ORL-1 receptor ligand
<220>
   <221> MISC_FEATURE
   <222> (1)..(17)
   <223> where X is an alpha helix promoter
<400> 20
   Phe Gly Gly Phe Thr Gly Ala Arg Lys Ser Ala Arg Lys Leu Xaa Asn
   1               5                   10                  15
   Gln
<210> 21
   <211> 17
   <212> PRT
   <213> Artificial Sequence
<220>
   <223> ORL-1 receptor ligand
<220>
   <221> MISC_FEATURE
   <222> (1)..(17)
   <223> where X is an alpha helix promoter
<400> 21
   Phe Gly Gly Phe Thr Gly Xaa Arg Lys Ser Xaa Arg Lys Leu Ala Asn
   1               5                   10                  15
   Gln
<210> 22
   <211> 17
   <212> PRT
   <213> Artificial Sequence
<220>
   <223> ORL-1 receptor ligand
<220>
   <221> MISC_FEATURE
   <222> (1)..(17)
   <223> where X is an alpha helix promoter
<400> 22
   Phe Gly Gly Phe Thr Gly Xaa Arg Lys Ser Ala Arg Lys Leu Xaa Asn
   1 5 10 15
   Gln
<210> 23
   <211> 17
   <212> PRT
   <213> Artificial Sequence
<220>
   <223> ORL-1 receptor ligand
<220>
   <221> MISC_FEATURE
   <222> (1)..(17)
   <223> where X is an alpha helix promoter
<400> 23
   Phe Gly Gly Phe Thr Gly Ala Arg Lys Ser Xaa Arg Lys Leu Xaa Asn
   1 5 10 15
   Gln
<210> 24
   <211> 17
   <212> PRT
   <213> Artificial Sequence
<220>
   <223> ORL-1 receptor ligand
<220>
   <221> MISC_FEATURE
   <222> (1)..(17)
   <223> where X is an alpha helix promoter
<400> 24
   Phe Gly Gly Phe Thr Gly Xaa Arg Lys Ser Xaa Arg Lys Leu Xaa Asn
   1 5 10 15
   Gln
<210> 25
   <211> 17
   <212> PRT
   <213> Artificial Sequence
<220>
   <223> ORL-1 receptor ligand
<220>
   <221> MISC_FEATURE
   <222> (1)..(17)
   <223> where X is an extended beta-strand promoter (comparative)
<400> 25
   Phe Gly Gly Phe Thr Gly Xaa Arg Lys Ser Ala Arg Lys Leu Ala Asn
   1               5                   10                  15
   Gln
<210> 26
   <211> 17
   <212> PRT
<213> Artificial Sequence
<220>
   <223> ORL-1 receptor ligand
<220>
   <221> MISC_FEATURE
   <222> (1)..(17)
   <223> where X is an extended beta-strand promoter (comparative)
<400> 26
   Phe Gly Gly Phe Thr Gly Ala Arg Lys Ser Xaa Arg Lys Leu Ala Asn
   1               5                   10                  15
   Gln
<210> 27
   <211> 17
   <212> PRT
   <213> Artificial Sequence
<220>
   <223> ORL-1 receptor ligand
<220>
   <221> MISC_FEATURE
   <222> (1)..(17)
   <223> where X is an extended beta-strand promoter (comparative)
<400> 27
   Phe Gly Gly Phe Thr Gly Ala Arg Lys Ser Ala Arg Lys Leu Xaa Asn
   1               5                   10                  15
   Gln
<210> 28
   <211> 17
   <212> PRT
   <213> Artificial Sequence
<220>
   <223> ORL-1 receptor ligand
<220>
   <221> MISC_FEATURE
   <222> (1)..(17)
   <223> where X is an extended beta-strand promoter (comparative)
<400> 28
   Phe Gly Gly Phe Thr Gly Xaa Arg Lys Ser Xaa Arg Lys Leu Ala Asn
   1 5 10 15
   Gln
<210> 29
   <211> 17
   <212> PRT
   <213> Artificial Sequence
<220>
   <223> ORL-1 receptor ligand
<220>
   <221> MISC_FEATURE
   <222> (1)..(17)
   <223> where X is an extended beta-strand promoter (comparative)
<400> 29
   Phe Gly Gly Phe Thr Gly Xaa Arg Lys Ser Ala Arg Lys Leu Xaa Asn
   1 5 10 15
   Gln
<210> 30
   <211> 17
   <212> PRT
   <213> Artificial Sequence
<220>
   <223> ORL-1 receptor ligand
<220>
   <221> MISC_FEATURE
   <222> (1)..(17)
   <223> where X is an extended beta-strand promoter (comparative)
<400> 30
   Phe Gly Gly Phe Thr Gly Ala Arg Lys Ser Xaa Arg Lys Leu Xaa Asn
   1 5 10 15
   Gln
<210> 31
   <211> 17
   <212> PRT
   <213> Artificial Sequence
<220>
   <223> ORL-1 receptor ligand
<220>
   <221> MISC_FEATURE
   <222> (1)..(17)
   <223> where X is an extended beta-strand promoter (comparative)
<400> 31
   Phe Gly Gly Phe Thr Gly Xaa Arg Lys Ser Xaa Arg Lys Leu Xaa Asn
   1 5 10 15
   Gln
<210> 32
   <211> 17
   <212> PRT
   <213> Artificial Sequence
<220>
   <223> ORL-1 receptor ligand
<220>
   <221> MISC_FEATURE
   <222> (1)..(17)
   <223> where X is alpha-methyl alanine
<400> 32
   Phe Gly Gly Phe Thr Gly Xaa Arg Lys Ser Ala Arg Lys Leu Ala Asn
   1 5 10 15
   Gln
<210> 33
   <211> 17
   <212> PRT
   <213> Artificial Sequence
<220>
   <223> ORL-1 receptor ligand
<220>
   <221> MISC_FEATURE
   <222> (1)..(17)
   <223> where X is alpha-methyl alanine
<400> 33
   Phe Gly Gly Phe Thr Gly Ala Arg Lys Ser Xaa Arg Lys Leu Ala Asn
   1               5                   10                  15
   Gln
<210> 34
   <211> 17
   <212> PRT
   <213> Artificial Sequence
<220>
   <223> ORL-1 receptor ligand
<220>
   <221> MISC_FEATURE
   <222> (1)..(17)
   <223> where X is alpha-methyl alanine
<400> 34
   Phe Gly Gly Phe Thr Gly Ala Arg Lys Ser Ala Arg Lys Leu Xaa Asn
   1               5                   10                  15
   Gln
<210> 35
   <211> 17
   <212> PRT
   <213> Artificial Sequence
<220>
   <223> ORL-1 receptor ligand
<220>
   <221> MISC_FEATURE
   <222> (1)..(17)
   <223> (1)..(17) where X is N-methyl alanine (comparative)
<400> 35
   Phe Gly Gly Phe Thr Gly Xaa Arg Lys Ser Ala Arg Lys Leu Ala Asn
   1               5                   10                  15
   Gln
<210> 36
   <211> 17
   <212> PRT
   <213> Artificial Sequence
<220>
   <223> ORL-1 receptor ligand
<220>
   <221> MISC_FEATURE
   <222> (1)..(17)
   <223> where X is N-methyl alanine (comparative)
<400> 36
   Phe Gly Gly Phe Thr Gly Ala Arg Lys Ser Xaa Arg Lys Leu Ala Asn
   1               5                   10                  15
   Gln
<210> 37
   <211> 17
   <212> PRT
   <213> Artificial Sequence
<220>
   <223> ORL-1 receptor ligand
<220>
   <221> MISC_FEATURE
   <222> (1)..(17)
   <223> where X is N-methyl alanine (comparative)
<400> 37
   Phe Gly Gly Phe Thr Gly Ala Arg Lys Ser Ala Arg Lys Leu Xaa Asn
   1               5                   10                  15
   Gln
<210> 38
   <211> 17
   <212> PRT
   <213> Artificial Sequence
<220>
   <223> ORL-1 receptor ligand
<220>
   <221> MISC_FEATURE
   <222> (1)..(17)
   <223> where X is alpha-methyl alanine; amidation at the C-terminus
<400> 38
   Phe Gly Gly Phe Thr Gly Xaa Arg Lys Ser Ala Arg Lys Leu Ala Asn
   1 5 10 15
   Gln
<210> 39
   <211> 17
   <212> PRT
   <213> Artificial Sequence
<220>
   <223> ORL-1 receptor ligand
<220>
   <221> MISC_FEATURE
   <222> (1)..(17)
   <223> where X is alpha-methyl alanine; amidation at the C-terminus
<400> 39
   Phe Gly Gly Phe Thr Gly Ala Arg Lys Ser Xaa Arg Lys Leu Ala Asn
   1 5 10 15
   Gln
<210> 40
   <211> 17
   <212> PRT
   <213> Artificial Sequence
<220>
   <223> ORL-1 receptor ligand
<220>
   <221> MISC_FEATURE
   <222> (1)..(17)
   <223> where X is alpha-methyl alanine; amidation at the C-terminus
<400> 40
   Phe Gly Gly Phe Thr Gly Ala Arg Lys Ser Ala Arg Lys Leu Xaa Asn
   1 5 10 15
   Gln
<210> 41
   <211> 17
   <212> PRT
   <213> Artificial Sequence
<220>
   <223> ORL-1 receptor ligand
<220>
   <221> MISC_FEATURE
   <222> (1)..(17)
   <223> where X is N-methyl alanine; amidation at the C-terminus (comparative)
<400> 41
   Phe Gly Gly Phe Thr Gly Xaa Arg Lys Ser Ala Arg Lys Leu Ala Asn
   1               5                   10                  15
   Gln
<210> 42
   <211> 17
   <212> PRT
   <213> Artificial Sequence
<220>
   <223> ORL-1 receptor ligand
<220>
   <221> MISC_FEATURE
   <222> (1)..(17)
   <223> where X is N-methyl alanine; amidation at the C-terminus (comparative)
<400> 42
   Phe Gly Gly Phe Thr Gly Ala Arg Lys Ser Xaa Arg Lys Leu Ala Asn
   1               5                   10                  15
   Gln
<210> 43
   <211> 17
   <212> PRT
   <213> Artificial Sequence
<220>
   <223> ORL-1 receptor ligand
<220>
   <221> MISC_FEATURE
   <222> (1)..(17)
   <223> where X is N-methyl alanine; amidation at the C-terminus (comparative)
<400> 43
   Phe Gly Gly Phe Thr Gly Ala Arg Lys Ser Ala Arg Lys Leu Xaa Asn
   1 5 10 15
   Gln
<210> 44
   <211> 17
   <212> PRT
   <213> Artificial Sequence
<220>
   <223> ORL-1 receptor ligand
<220>
   <221> MISC_FEATURE
   <222> (1)..(17)
   <223> where X is alpha-methyl alanine; amidation at the C-terminus
<400> 44
   Phe Gly Gly Phe Thr Gly Xaa Arg Lys Ser Xaa Arg Lys Leu Ala Asn
   1 5 10 15
   Gln
<210> 45
   <211> 9
   <212> PRT
   <213> Homo sapiens
<400> 45
   Ala Arg Lys Ser Ala Arg Lys Leu Ala
   1 5
<210> 46
   <211> 9
   <212> PRT
   <213> Artificial Sequence
<220>
   <223> ORL-1 receptor ligand
<220>
   <221> MISC_FEATURE
   <222> (1)..(9)
   <223> where X is unknown or other
<400> 46
   Ala Arg Lys Xaa Ala Arg Lys Xaa Ala
   1 5
<210> 47
   <211> 4
   <212> PRT
   <213> Artificial Sequence
<220>
   <223> ORL-1 receptor ligand
<220>
   <221> MISC_FEATURE
   <222> (1)..(1)
   <223> where Xaa is an alpha helix promoter or an extended beta strand promoter (comparative)
<220>
   <221> MISC_FEATURE
   <222> (2)..(3)
   <223> where Xaa is any basic amino acid or basic amino acid analog
<220>
   <221> MISC_FEATURE
   <222> (4)..(4)
   <223> where Xaa is any amino acid or amino acid analog
<400> 47
   Xaa Xaa Xaa Xaa
   1
<210> 48
   <211> 7
   <212> PRT
   <213> Artificial Sequence
<220>
   <223> ORL-1 receptor ligand
<220>
   <221> MISC_FEATURE
   <222> (1)..(7)
   <223> where Xaa is an alpha helix promoter
<400> 48
   Ala Arg Lys Ser Xaa Arg Lys
   1 5
<210> 49
   <211> 7
   <212> PRT
   <213> Artificial Sequence
<220>
   <223> ORL-1 receptor ligand
<220>
   <221> MISC_FEATURE
   <222> (1)..(7)
   <223> where Xaa is an alpha helix promoter
<400> 49
   Xaa Arg Lys Ser Ala Arg Lys
   1               5
<210> 50
   <211> 7
   <212> PRT
   <213> Artificial Sequence
<220>
   <223> ORL-1 receptor ligand
<220>
   <221> MISC_FEATURE
   <222> (1)..(7)
   <223> where Xaa is an alpha helix promoter
<400> 50
   Xaa Arg Lys Ser Xaa Arg Lys
   1               5
<210> 51
   <211> 13
   <212> PRT
   <213> Artificial Sequence
<220>
   <223> ORL-1 receptor ligand
<220>
   <221> MISC_FEATURE
   <222> (1)..(13)
   <223> where Xaa is an alpha helix promoter
<400> 51
   Phe Gly Gly Phe Thr Gly Ala Arg Lys Ser Xaa Arg Lys
   1               5                   10
<210> 52
   <211> 13
   <212> PRT
   <213> Artificial Sequence
<220>
   <223> ORL-1 receptor ligand
<220>
   <221> MISC_FEATURE
   <222> (1)..(13)
   <223> where Xaa is an alpha helix promoter
<400> 52
   Phe Gly Gly Phe Thr Gly Xaa Arg Lys Ser Ala Arg Lys
   1               5                   10
<210> 53
   <211> 13
   <212> PRT
   <213> Artificial Sequence
<220>
   <223> ORL-1 receptor ligand
<220>
   <221> MISC_FEATURE
   <222> (1)..(13)
   <223> where Xaa is an alpha helix promoter
<400> 53
   Phe Gly Gly Phe Thr Gly Xaa Arg Lys Ser Xaa Arg Lys
   1               5                   10
<210> 54
   <211> 13
   <212> PRT
   <213> Artificial Sequence
<220>
   <223> ORL-1 receptor ligand
<220>
   <221> MISC_FEATURE
   <222> (1)..(13)
   <223> where X is alpha-methyl alanine; amidation at the C-terminus
<400> 54
   Phe Gly Gly Phe Thr Gly Ala Arg Lys Ser Xaa Arg Lys
   1               5                   10
<210> 55
   <211> 13
   <212> PRT
   <213> Artificial Sequence
<220>
   <223> ORL-1 receptor ligand
<220>
   <221> MISC_FEATURE
   <222> (1)..(13)
   <223> where Xaa is alpha-methyl alanine; amidation at the C-terminus
<400> 55
   Phe Gly Gly Phe Thr Gly Xaa Arg Lys Ser Ala Arg Lys
   1               5                   10
<210> 56
   <211> 13
   <212> PRT
   <213> Artificial Sequence
<220>
   <223> ORL-1 ligand
<220>
   <221> MISC_FEATURE
   <222> (1)..(13)
   <223> where Xaa is alpha-methyl alanine; amidation at the C-terminus
<400> 56
   Phe Gly Gly Phe Thr Gly Xaa Arg Lys Ser Xaa Arg Lys
   1               5                   10